# EUROPEAN PATENT SPECIFICATION

(11) **EP 4 449 120 B1**
(45) Date of publication and mention of the grant of the patent: **13.08.2025**
(21) Application number: 22829829.5
(22) Date of filing: 14.12.2022
(51) Int. Cl.: G01N 33/537, G01N 33/539, G01N 33/68

(54) **PROTEIN STABILITY ASSAY**
PROTEINSTABILITÄTSTEST
TEST DE STABILITÉ DES PROTÉINES

(30) Priority: 15.12.2021 GB 202118173
(43) Date of publication of application: 23.10.2024
(60) Divisional application: 25179067.1
(73) Proprietor: Medicines Discovery Catapult Limited, Macclesfield, Cheshire SK10 4ZF (GB)
(72) Inventor: MAIN, Martin, Macclesfield, Cheshire SK10 4ZF (GB); DAUBNER, Gerrit, 69123 Heidelberg (DE); VINCENT, John, Macclesfield, Cheshire SK10 4ZF (GB); BURNHAM, Matthew, Macclesfield, Cheshire SK10 4ZF (GB); HART, Philippa Jayne, Macclesfield, Cheshire SK10 4ZF (GB)
(74) Representative: HGF
(86) International application number: PCT/GB2022/053220
(87) International publication number: WO 2023/111554

(56) References cited:
- EP-A1- 0 770 876
- WO-A1-2021/098775
- ZHANG XIAOLEI ET AL: "Solvent-Induced Protein Precipitation for Drug Target Discovery on the Proteomic Scale", ANALYTICAL CHEMISTRY, vol. 92, no. 1, 3 December 2019 (2019-12-03), US, pages 1363 - 1371, XP055813477, ISSN: 0003-2700, DOI: 10.1021/acs.analchem.9b04531

## Description

### FIELD OF THE INVENTION

The invention relates to methods for identifying whether a protein target and compound of interest bind to one another. The methods may be useful in applications such as drug discovery.

### INTRODUCTION

Detecting or confirming that a putative ligand binds to a target protein is important in many areas of medicine and biology. Drug discovery by pharmaceutical companies is a major area that utilises assays designed to detect this 'target engagement' by chemical compounds, or by other modalities of therapeutic molecule, designed to bind to the target protein and have a resultant therapeutic effect. These assays can be used for initial screening to find putative ligands from large chemical libraries, to optimise a chemical compound to improve its drug-like properties, or to elucidate the mechanism of action of a compound or other molecule.

Target engagement assays that have been developed in the past include methods based on detection of the thermodynamic stabilisation of a target protein structure that can occur upon binding of a ligand. A previously developed thermal denaturation assay, also known as a thermal shift assay or differential scanning fluorimetry (DSF; Niesen et al., (2007); Nature Protocols 2, 2212-2221), uses a purified target protein and a fluorescent dye to determine the increase in the temperature required to unfold the target protein upon binding of a ligand to the target protein.

While methods are established to determine target engagement in purified samples of target protein or protein fragments, it is important to confirm target engagement in a cellular context where the target protein will be in a more representative state. Thus, in the cell, the target protein will be its native (full length) form, with appropriate post-translational modifications, and will interact with associated interacting proteins. The cellular thermal stability assay (CETSA; Molina et al., (2013); Science 341, 84-87) was developed to address these points. In this case, a thermal stimulus is applied to cells containing the target protein to trigger protein denaturation, and this is followed by separation of native folded protein and denatured proteins using centrifugation. Finally, a detection step in the assay measures the proportion of the target protein in its native form, with a change detected in ligand-bound samples indicating target-engagement.

However, these methods are not ideal, particularly for applications where large number of samples need to be screened, such as a high-throughput screen or lead optimisation campaign: Methods utilising a purified protein target may be difficult to achieve for proteins that are difficult to purify at scale and with suitable conformations; and as described above can present the target protein in a form that is less representative of its true cellular state. Methods requiring the separation of samples into native and denatured protein fractions are cumbersome to perform at large scale. Furthermore, thermal stability methods require a precise control of sample temperature for precise durations which can be difficult to achieve at scale or require transfer between vessels. Stabilisation assay methods which use alternatives to thermal denaturation may provide novel insights into ligand-target interactions. Therefore, there is a need for a binding assay that offers a sensitive and robust detection of ligand target engagement in a simple homogeneous assay format amenable to screening workflows.

It is recognised that commonly used methods for investigating target engagement see for instance EP 0770 876 and WO 2021 098775, are laborious (particularly in terms of the requirement for multiple pipetting steps associated with the transfer of samples between different vessels) and associated with high rates of sample consumption.

### SUMMARY OF THE INVENTION

The present invention provides a protein stability assay (PSA).

In a first aspect, the invention provides a method of identifying whether a protein target and a compound of interest bind to one another, the method comprising, in order, the steps of:
i) exposing the protein target to the compound of interest for a time sufficient to allow binding of the compound of interest to the protein target;
ii) contacting the protein target with a chemical denaturing agent to produce a denaturing mixture;
iii) assaying the products of the denaturing mixture to determine the proportion of the protein target in its folded state and/or the proportion of the protein target in its denatured state;

wherein a change in the proportion of the protein target in its folded state as compared to a suitable control indicates that the protein target and compound of interest bind to one another, and wherein step ii) of the method is conducted at a substantially constant temperature;
wherein steps ii) and iii) are conducted in the same vessel; and wherein the chemical denaturing agent comprises at least one of: an acid; an alcohol; and a ketone.

The methods of the first aspect of the invention are useful in identifying compounds that bind to a protein target by directly assessing the impact of the compound on stability of the protein target in response to chemical denaturing conditions.

The binding of a compound to its target protein can lead to stabilisation (or destabilisation) of proteins that are associated with the target protein. So, measuring the stability of the associated protein can be used as a surrogate for protein-target binding and/or can be used to identify associations of proteins within a pathway/protein complex. A similar approach has been used with the CETSA thermal protein stabilising approach (Savitski et al. Science. 346 (6205): 2014; DOI: 10.1126/science.1255784).

Accordingly, the methods disclosed herein may also be used to identify compounds that bind to a protein target "indirectly" by assessing the impact of the compound on the stability of an associated protein that forms a complex with the protein target.

Accordingly, in a second aspect, disclosed herein but not part of the claimed invention, is a method of identifying whether a protein target and a compound of interest bind to one another, the method comprising, in order, the steps of:
i) exposing the protein target, in the presence of an associated protein that is part of a complex with the protein target, to the compound of interest for a time sufficient to allow binding of the compound of interest to the protein target;
ii) contacting the associated protein with a chemical denaturing agent to produce a denaturing mixture;
iii) assaying the products of the denaturing mixture to determine the proportion of the associated protein in its folded state and/or the proportion of the associated protein in its denatured state;
wherein a change in the proportion of the associated protein in its folded state as compared to a suitable control indicates that the protein target and compound of interest bind to one another, and wherein step ii) of the method is conducted at a substantially constant temperature.

In the description that follows, except for where the context requires otherwise, references to a "method of the invention" should be taken as applying to both methods of the first aspect of the invention and methods of the second aspect. Furthermore, considerations set out in respect of methods of the first aspect of the invention should be taken as applicable to methods in respect of the second aspect unless the context requires otherwise, and *vice versa.*

### DETAILED DESCRIPTION OF THE INVENTION

The methods of the invention make use of the capacity of compounds that bind to proteins to alter the stability of the bound proteins (or proteins associated with bound proteins) in response to denaturing conditions. Binding may increase or decrease the stability of the protein, depending on the nature of the protein and/or the compound of interest. The detectable change in the stability of the protein that occurs provides the basis for many well-known protein target engagement assays. However, the methods of the invention incorporate a number of important differences as compared to those methods that have previously been described in the prior art.

In particular, the methods of the invention use chemical agents to bring about the required denaturing and, in contrast to the prior art, do not require the mixtures used to undergo significant temperature changes during the course of the method. Instead, the denaturation required by the methods of the invention is able to be achieved while the reagents are maintained at a substantially constant temperature.

By avoiding the requirement to heat mixtures in order to achieve a desired level of denaturation, as is the case for previously published techniques, the methods of the present invention are able to avoid the need for the reagents used in the methods to be transferred between different vessels. This is required in prior art techniques, as the vessels used to heat samples are not suitable for further assaying steps, and those vessels well adapted to assaying do not allow efficient and reproducible transfer of heat.

These changes adopted in the methods of the invention reduce complexity involved in practicing such methods. This confers important advantages that are not offered by prior art techniques. In particular, the methods of the invention are well suited to use in high throughput assays for the investigation of target engagement. Previously reported methods of investigating target engagement have not been well adapted to high throughput use in this manner. Generally, previously published methods have required at least one step in which samples are transferred between vessels. It is recognised that this requirement of earlier assays has an adverse impact on their speed and efficiency, and that this, in turn, imposes undesirable limitations on the process of drug discovery. Previously published methods often also required separation of soluble from insoluble proteins, for example using a rigorous centrifugation step, which necessitates at least one step in which samples are transferred between vessels.

The assay of the present system can be configured for high throughput. This also allows the system to be employed as a toolbox to test different denaturing agents in parallel to identify those that are best suited for use with the target protein of interest.

The present invention will now be further described with reference to the following paragraphs, which may be useful in understanding certain embodiments of the invention.

### DEFINITIONS

As used herein and unless stated otherwise, it is to be understood that the term "about" when referring to a value (e.g. amount of an ingredient or percentage) is used synonymously with the term "approximately". Illustratively and unless stated otherwise, the use of the term "about" indicates values slightly outside the cited criteria values, for example ±15%, ± 10% ± 8%, ± 5% or conveniently ± 2%. Such values are thus encompassed by the scope of the claims reciting the terms "about" or approximately".

### Steps conducted "at a substantially constant temperature"

The methods of the invention require one or more of the steps of the method to be conducted "at a substantially constant temperature".

In particular, at least step ii) of a method in accordance with the present invention is conducted at a substantially constant temperature. That said, some or all of the remaining steps of a method of the invention may also be conducted at a substantially constant temperature. Accordingly, in addition to step ii), other steps of the method of the invention, for example, step iii) and/or step i) of the method may also be conducted at a substantially constant temperature. Thus, in a suitable method of the invention, steps i) and ii) are conducted at a substantially constant temperature. In a suitable embodiment of a method of the invention, steps ii) and iii) are conducted at a substantially constant temperature. Or in a suitable embodiment of a method of the invention, each of steps i), ii) and iii) are conducted at a substantially constant temperature.

Suitably, in each of the above situations where the method is conducted at a substantially constant temperature in two or more steps of the method, the method is conducted at substantially the same constant temperature in each of these steps.

Thus, in an embodiment of the first aspect, the invention provides a method of identifying whether a protein target and a compound of interest bind to one another, the method comprising, in order, the steps of:
i) exposing the protein target to the compound of interest for a time sufficient to allow binding of the compound of interest to the protein target;
ii) contacting the protein target with a chemical denaturing agent to produce a denaturing mixture;
iii) assaying the products of the denaturing mixture to determine the proportion of the protein target in its folded state and/or the proportion of the protein target in its denatured state;
wherein a change in the proportion of the protein target in its folded state as compared to a suitable control indicates that the protein target and compound of interest bind to one another, and wherein steps i), ii) and iii) of the method are conducted at a substantially constant temperature.

Suitably the method is carried out in the same vessel. Such vessel could be a well of a multiwell plate (such as a 96-, 384- or 1536-well plate). The use of a multiwell plate allows the system to be configured for high throughput screening.

Disclosed herein is a method of identifying whether a protein target and a compound of interest bind to one another, the method comprising, in order, the steps of:
i) exposing the protein target, in the presence of an associated protein that is part of a complex with the protein target, to the compound of interest for a time sufficient to allow binding of the compound of interest to the protein target;
ii) contacting the associated protein with a chemical denaturing agent to produce a denaturing mixture;
iii) assaying the products of the denaturing mixture to determine the proportion of the associated protein in its folded state and/or the proportion of the associated protein in its denatured state;
wherein a change in the proportion of the associated protein in its folded state as compared to a suitable control indicates that the protein target and compound of interest bind to one another, and wherein steps i), ii) and iii) of the method are conducted at a substantially constant temperature.

Suitably the method is carried out in the same vessel. Such vessel could be a well of a multiwell plate (such as a 96, 384 or 1536 well plate). The use of a multiwell plate allows the system to be configured for high throughput screening.

For the purposes of the present disclosure, references to a step, or steps, of a method of the invention being conducted at substantially constant temperature should be construed as requiring that the temperature of the reaction constituents does not change substantially during the recited period. For example, the temperature may not change by more than 10°C,may not change by more than 9°C, may not change by more than 8°C, may not change by more than 7°C, may not change by more than 6°C, may not change by more than 5°C, may not change by more than 4°C, may not change by more than 3°C, may not change by more than 2°C, or may not change by more than 1°C during the recited period.

In the case of a method of the invention in which multiple steps of the method are conducted at a substantially constant temperature, each step may be conducted at the same substantially constant temperature. Thus, in the case of a method of the invention in which steps ii) and i) are conducted at a substantially constant temperature, each of step ii) and step i) may be conducted at the same substantially constant temperature. This substantially constant temperature may be maintained throughout the full duration of steps ii) and i). In the case of a method of the invention in which steps ii) and iii) are conducted at a substantially constant temperature, each of step ii) and step iii) may be conducted at the same substantially constant temperature. This substantially constant temperature may be maintained throughout the full duration of steps ii) and iii). And in the case of a method of the invention in which steps i), ii) and iii) are conducted at a substantially constant temperature, each of step ii) and step i) may be conducted at the same substantially constant temperature, or each of step ii) and step iii) may be conducted at the same substantially constant temperature, or each of step i) and step iii) may be conducted at the same substantially constant temperature. This substantially constant temperature may be maintained throughout the full duration of steps i), ii) and iii).

In a suitable method in accordance with the invention, each of steps i), ii) and iii) of the method may be conducted at about 20°C.

In a suitable method in accordance with the invention, each of steps i), ii) and iii) of the method may be conducted at about 25°C.

In a suitable method in accordance with the invention, each of steps i), ii) and iii) of the method may be conducted at about 30°C.

In a suitable method in accordance with the invention, each of steps i), ii) and iii) of the method may be conducted at about 37°C.

In a suitable method in accordance with the invention, steps i) and iii) of the method may be carried out at a temperature of about 30°C and step ii) of the method may be conducted at about 37°C.

As referred to above, the use of substantially constant temperatures (which may be ambient temperature, as discussed below) is in contrast to prior art techniques that rely upon increased temperature sufficient to denature protein targets. Such techniques typically involve multiple temperature changes over the course of the method. In addition to increasing temperature during a step to denature the protein target, such methods also frequently involve a rapid cooling step (often with reactions mixtures being placed on ice, or the like) at the end of the heating period. Other systems that do not rely on heat denaturation often also require changes in temperature, e.g. a rapid cooling step (often with reactions mixtures being placed on ice, or the like). This is associated with a need to physically move reaction mixtures and/or to transfer them between different vessels. It will be appreciated that these requirements significantly complicate the process of performing such prior art methods, and limit their capacity for automation. In addition, certain prior art methods require separation of the soluble and insoluble components of a reaction mixture, typically using a rigorous centrifugation step that either includes or follows a temperature shift requirement and/or is associated with a need to physically move reaction mixtures and/or to transfer them between different vessels. By rigorous centrifugation we mean centrifugation at a spin speed and duration sufficient to separate soluble from insoluble components in the mixture/liquor, rather than a short pulse spin (e.g. 1,000 rpm for a few seconds to a minute) designed solely to move the liquid to the base of a vessel or settle the mixture. A rigorous centrifugation includes one that would results in sedimentation of particulate matter, e.g. cause formation of a cellular debris pellet.

### Methods conducted without heating

The inventors have found that it is not necessary to heat the constituents used in the methods of the invention (for example in the form of a denaturing mixture) in order to achieve an extent of protein denaturation that is sufficient to allow cases in which a compound of interest binds to a protein target to be distinguished from cases in which such binding does not occur. Instead, the entire extent of denaturing required may be provided by the chemical denaturing agent used. This is a surprising departure from the prior art, and leads to a number of notable advantages offered by the methods of the invention.

The inventors' finding enables some or all of the steps of a method of the invention to be conducted without a heating step to a temperature sufficient to cause protein denaturation.

This ability to forego heating during the period when denaturing of the target protein can occur is in contrast to methods of the prior art, which typically use elevated temperatures to either bring about denaturing, to accelerate the denaturing process, or to complete denaturation.

Accordingly, and surprisingly, in a suitable embodiment of a method of the invention, step ii) may be conducted without heating the denaturing mixture. Heating may also be omitted in respect of some or all other steps of the method of the invention. For example, step iii) and/or step i) of the method may also be conducted without heating. In a suitable method of the invention, steps i) and ii) are conducted without heating. In a suitable embodiment of a method of the invention, steps ii) and iii) are conducted without heating. Or in a suitable embodiment of a method of the invention, each of steps i), ii) and iii) are conducted without heating.

The ability to practice the methods of the present invention without heating considerably simplifies their practice as compared to the methods of the prior art. Merely by way of example, they avoid the need for certain steps of the method to be performed in specialist vessels chosen for their ability to conduct heat. Such vessels (typically "PCR plates") have advantageous properties in terms of their thermal conductance (ensuring that heat is evenly and reproducibly transferred to their contents) but are not well adapted to use in other steps of the methods of the invention, in particular the assaying of step iii). PCR plates often lack rigidity (which in turn leads to non-uniform signal generation between different areas of the plate) and are not the correct shape to be used in automated plate readers, in that the wells are v-shaped instead of flat. These failings make it necessary to transfer the reactants from the vessel in which heating to effect denaturation occurs to a separate vessel for assaying and/or analysis. By avoiding the need for heating, the methods of the invention remove the need to transfer samples between different vessels, thus simplifying methods in accordance with this embodiment. This simplification also facilitates the use of such methods of the invention in high throughput applications.

Suitably, step ii) of the method of the invention is carried out without an increase in heating designed to effect protein denaturation.

Suitably, step ii) of the method of the invention is carried out at a temperature which does not result in significant protein denaturation.

### Methods conducted at ambient temperature

Consistent with the ability to avoid the requirement for heating (to effect denaturation), and in keeping with the use of a substantially constant temperature, the inventors have found that some or all of the steps of a method of the invention may be practiced at ambient temperature.

In particular, at least step ii) of a method in accordance with the present invention may conducted at ambient temperature. Alternatively, or additionally, some or all other steps of the invention may also be conducted at ambient temperature. For example, step iii) and/or step i) of a method of the invention may also be conducted at ambient temperature. Thus, step ii) and step i) of a method of the invention, or step ii) and step iii) of a method of the invention, may be conducted at ambient temperature. Suitably each of steps i), ii) and iii) of a method of the invention may be conducted at ambient temperature. When a method of the invention comprises further steps, some or all of these further steps may also be conducted at ambient temperature.

The expectation, established within the prior art, that increased temperature is required to practice an effective denaturing step, makes it particularly surprising that embodiments of the methods of the invention in which the step ii) is conducted at ambient temperature are effective.

In addition to being surprising, such embodiments offer advantages in terms of their simplicity, in that there is no need to control the temperature of the reaction during the denaturing step. Furthermore, such methods offer the advantage (discussed elsewhere) that they do not require the use of separate vessels used to heat the reactants, thus reducing or avoiding the need to transfer the constituents during the course of the method of the invention.

For the purposes of the present disclosure, ambient temperature may be regarded as corresponding to room temperature. In particular, step ii) (and step iii) and/or any other selected step) of a method of the invention may be conducted at a temperature of between approximately 18°C and 22°C. For example, step ii) and any other selected steps of the method may be conducted at a temperature of between approximately 19°C and 21°C. Steps of a method of the invention practiced at ambient temperature may be conducted at a temperature of approximately 20°C.

In a suitable method in accordance with the invention, step ii) of the method may be conducted at a temperature of about 37°C, or less. Suitably, steps iii) and/or i) of a method in accordance with the invention may also be conducted at a temperature of about 37°C, or less.

In a suitable method in accordance with the invention, steps i) and iii) of the method may be carried out at ambient temperature and step ii) of the method may be conducted at a temperature of about 30°C.

In a suitable method in accordance with the invention, step i) of the method may be carried out at a temperature of about 30°C and steps ii) and/or iii) of a method in accordance with the invention may be conducted at ambient temperature.

In a suitable method in accordance with the invention, step i) of the method may be carried out at ambient temperature and steps ii) and/or iii) of a method in accordance with the invention may be conducted at a temperature of about 30°C.

In a suitable method in accordance with the invention, steps i) and iii) of the method may be carried out at a temperature of about 30°C and step ii) of the method may be conducted at ambient temperature.

In a suitable method in accordance with the invention, steps i) and iii) of the method may be carried out at ambient temperature and step ii) of the method may be conducted at a temperature of about 37°C.

In a suitable method in accordance with the invention, step i) of the method may be carried out at a temperature of about 37°C and steps ii) and/or iii) of a method in accordance with the invention may be conducted at ambient temperature.

In a suitable method in accordance with the invention, step i) of the method may be carried out at ambient temperature and steps ii) and/or iii) of a method in accordance with the invention may be conducted at a temperature of about 37°C.

In a suitable method in accordance with the invention, steps i) and iii) of the method may be carried out at a temperature of about 37°C and step ii) of the method may be conducted at ambient temperature.

In a suitable method in accordance with the invention, each of steps i), ii) and iii) of the method may be conducted at ambient temperature.

For the avoidance of doubt, the considerations regarding temperature set out above should be taken as relating to the temperature of the denaturing mixture during step ii), and to other appropriate mixtures or constituents, *mutatis mutandis,* in the event that other steps of the method are to be practiced at ambient temperature.

### Protein targets

The methods of the invention may be used to investigate binding of compounds of interest to any protein target. Typically, the protein target may be an intracellular protein. However, the methods of the invention may also be used to identify binding in respect of extracellular proteins such as secreted proteins, or membrane proteins.

A protein target may be a biologically active protein. Merely by way of example, a suitable protein target may be selected from the group consisting of: an intracellular signalling protein; an enzyme (such as an intracellular enzyme); a structural protein; a transcription factor; a receptor protein; and a cytokine or growth factor.

Other suitable protein target may be selected from the group consisting of: an enzyme including kinase, phosphatase, protease, hydrolase, dehydrogenase, synthase, lipase, ligase; A growth factor receptor, including a receptor tyrosine kinase; an intracellular proteins, such as Bcl family, nuclear proteins, scaffold proteins, transcription factors and mitochondrial proteins; a membrane protein, including GPCR, Ion Channel, Transporter, Integrin; and a secreted protein such as a cytokine, chemokine or growth factor.

Since they do not require a heating step, the methods of the present invention may be particularly useful in the context of investigating binding of compounds of interest to target proteins that are sensitive to heat or modified by heat. In accordance with this embodiment, a target protein for use in a method of the invention may be a heat shock protein.

The protein target may be a protein that it is wished to drug, in which case the compound of interest may be a putative drug for the target protein, as discussed further below.

### Associated proteins

Described herein is a method for indirectly investigate the binding of a compound of interest to a protein target, by assessing a change in the stability of an "associated protein" that is part of a complex with the protein target. This opportunity has been reported in the CETSA field when researchers have compared a CETSA-Mass Spec assay run on cells incubated with compound, with lysate incubated with compound. For example, Savitski et al. (Science. 346 (6205): 2014; DOI: 10.1126/science.1255784) discussed the associated-protein results.

The understanding is that protein complexes are intact in the whole-cell experiment, but disaggregated in lysate. Thus, engagement of compounds of interest with certain protein targets may have an impact upon the resistance to denaturation of other proteins associated with the target protein, in addition or as an alternative to changing the ability of the target protein to resist denaturation.

As set out above, in such embodiments the protein target and associated protein form part of a complex. The protein target and associated protein may be directly linked to one another within the complex, or may be indirectly linked within the complex.

### Compounds of interest

A compound of interest will generally be a putative binding partner of the protein target.

Suitably, a compound of interest may be selected from the group consisting of: a small molecule compound that binds to the target and modulates the functional activity of the protein target; a small molecule compound that binds to the protein target, but does not modulate functional activity; a PROTAC compound or a compound that drives protein degradation; and antibody or other protein-binding affinity tool.

A compound of interest may be a putative modulator of activity of the protein target. For example, the compound of interest may be a putative inhibitor, a putative antagonist, or a putative negative modulator of the protein target. Alternatively, the compound of interest may a putative activator, a putative agonist, or a putative positive modulator of the protein target.

Without detracting from the above, it will be recognised that a suitable compound of interest may equally be a compound that is capable of binding to the target protein without functional effect. Merely by way of example, compounds of interest in accordance with this embodiment of the invention may include compounds that can act as target engaging portions of bifunctional molecules that interact with further natural or artificial binding partners. Examples of such bifunctional molecules may include those that engage with intracellular or extracellular systems, such as "proteolysis targeting chimeras" (PROTACS) that interact with the ubiquitin-proteosome system. Thus, the methods of the invention may be useful in identifying a compound of interest as suitable (or unsuitable) for use within such applications, for example as a target-binding portion of a PROTAC or other such bifunctional molecule.

A compound of interest may have any chemical nature. Merely by way of example, a compound of interest may be a small molecule, or may be a large molecule. Suitably the compound of interest may be a biological molecule, such as a protein, e.g. an antibody or a peptide.

The compound of interest may be part of a compound library.

The compound of interest may be a temperature sensitive compound. Since the methods of the invention do not require a step in which the reagents are subjected to increased temperature, they are well suited to investigating binding to protein targets by compounds of interest that may be damaged or rendered inactive by exposure to heat.

### Binding of a protein target and compound of interest

Binding, for the purposes of the present invention, may be considered to encompass any interaction between a protein target and compound of interest that alters the stability of the protein target.

In a suitable embodiment, the interaction is a non-covalent interaction between the protein target and a compound of interest. In an alternative embodiment, the interaction is a covalent interaction.

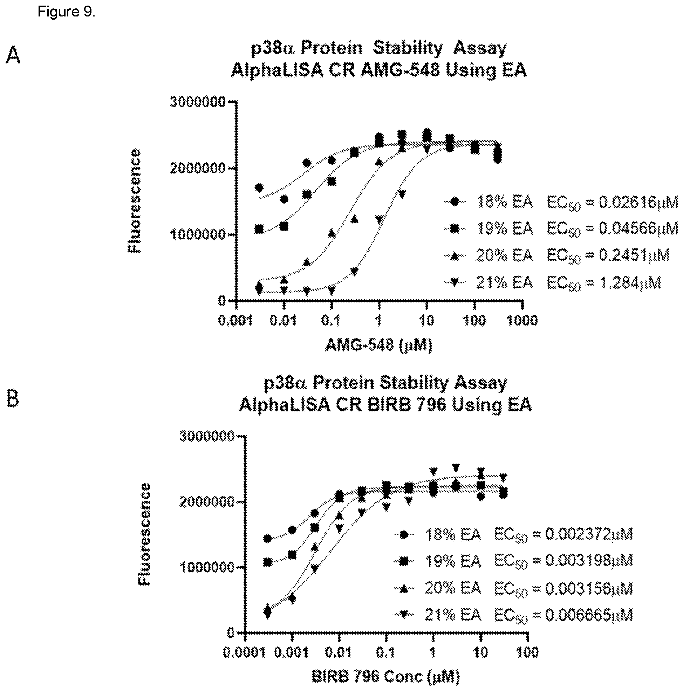

The binding between a protein target and a compound of interest may be reversible binding. The use of the methods of the invention in such an embodiment is illustrated in the results set out in Figure 9A.

Alternatively, the binding between a protein target and a compound of interest may be irreversible binding. The suitability of methods of the invention for use in such an embodiment is illustrated in the results set out in Figure 9B.

As mentioned elsewhere, binding of a compound of interest to a protein target may stabilise the protein target or an associated protein, thus rendering the protein target or associated protein more resistant to denaturation. Alternatively, binding of a compound of interest to a protein target may destabilise the protein target or an associated protein, thus rendering the protein target or associated protein more sensitive to denaturation.

### Exposing the protein target to a compound of interest

The methods rely upon exposing the protein target and compound of interest to one another for sufficient time and under conditions that allow binding between these two entities to occur. It will be appreciated that, such binding will not always take place, particularly in cases where the protein target and compound of interest do not interact with one another. However, such circumstances are still informative to a person practicing the invention, as they may indicate that the compound of interest is not suitable for use to bind to the protein target, and thus modulate its activity.

It will be appreciated that the protein stability assay method of the invention (e.g. as in the first aspect of the invention and second aspect) when conducted on a cell lysate will allow determination of compound:target binding; however, the use of whole cells ('intact cell' version of protein stability assay) rather than a lysate of cells will allow the user to measure cell penetration as well as compound:target binding. In this way, the methods of the invention can be employed to determine whether a test compound is capable or incapable of penetrating the cell, and/or reaching the target protein of interest.

A method of the invention may be practiced in such a manner to allow the generation of a concentration-response curve in respect of the compound of interest. The skilled person will be aware of arrangements that may be used to achieve this. Merely by way of example, the method may be practiced by parallel processing of a plurality of samples, wherein the compound of interest is provided to separate samples at different concentrations. The separate samples may be substantially identical to one another, other than with respect to the concentration of the compound of interest provided. As the method can be carried out in a standard multiwell plate (e.g. 24-well, 48-well, 96-well, 384-well, 1536-well or a greater number of well plate; e.g. 3456- or 9600-well) the different concentrations of a test compound (compound of interest) can be in separate wells of the same plate.

A suitable period of time may be selected with respect to a number of factors. Suitable factors may relate to the protein target, including whether or not the protein target is present within an intact biological cell (in which case the period of time may be sufficient to allow the compound of interest to penetrate the cell membrane), the location of a protein target within a cell, and the relative abundance of the protein target. Suitable factors may also relate to the compound of interest, including physicochemical properties of the compound of interest, such as the size or solubility of the compound of interest, and whether the compound is actively transported across the cell membrane.

In a suitable embodiment of a method of the invention, the protein target is in an intact cell when exposed to the compound of interest.

Alternatively, the protein target may suitably be present in a cell lysate when exposed to the compound of interest.

In a method of the invention in which the protein target is provided in an intact cell, an amount of a protein target sufficient to allow the practice of the method of the invention may be provided by providing a pre-determined number of cells that express the protein target in step i).

Suitably, in the case of a method of the invention practiced in a 384 well plate, or other multiwell plate, the pre-determined number of cells may be at least 1,000 cells, for example between about 1,000 and 100,000 cells per well of a 384-well plate, such as approximately 50,000 cells per well. The skilled person will appreciate that these numbers may be varied, *mutatis mutandis,* in the case that a vessel of a different size is used.

In a method of the invention in which the protein target is provided in a cell lysate, an amount of a protein target sufficient to allow the practice of the method of the invention may be provided by providing a pre-determined amount of a cell lysate in step i).

In the case of a method of the invention practiced in a 384-well plate, the pre-determined amount of a cell lysate may be around 8µg, for example between about 2µg and 30µg, such as between about 5µg and 10µg per well of a 384-well plate. The skilled person will appreciate that these amounts may be varied, *mutatis mutandis,* in the case that a vessel of a different size is used.

In a suitable embodiment of a method of the invention, the protein target is provided in step i) at a concentration of between 0.01 µg/µl of cell lysate and 25 µg/µl of cell lysate. For example, the protein target may be provided in step i) at a concentration of between 0.5 µg/µl of cell lysate and 10 µg/µl of cell lysate, or at a concentration of between 1 µg/µl of cell lysate and 5 µg/µl of cell lysate. Suitably, the protein target is provided in step i) at a concentration of between 2 µg/µl of cell lysate and 4 µg/µl of cell lysate
In principle, the assay can work at any compound concentration. The assay can tolerate 10% DMSO so one could test at a very high drug concentration.

In a suitable embodiment of a method of the invention, the compound of interest is provided in step i) at a concentration of up to 100 mM. The compound of interest may be provided in step i) at a concentration of up to 100 µM. The compound of interest may be provided in step i) at a concentration as low as 1nM. For example, the compound of interest may be provided in step i) at a concentration of between about 1nM and about 100 mM. Suitably, the compound of interest may be provided in step i) at a concentration of about 100nM to 10 µM. The skilled person will be able to select appropriate concentration as required (with high concentrations more likely to be preferred in the case of small molecule fragments), and screen these for effectiveness using the methods of the invention.

Suitably, the protein target is exposed to the compound of interest in step i) for a period of at least 5 minutes (such as at least 5, 10, 15, 20, 25, 30, 35, 40, 45, 50, 55 or 60 minutes). For example, the protein target may be exposed to the compound of interest in step i) for a period of at least 0.5 hours, for a period of at least 1 hour, or for a period of at least 2 hours. The protein target may even be exposed to the compound of interest in step i) of the method of the invention for at least 5 hours, at least 8 hours, or at least 12 hours.

Merely by way of example, the protein target may suitably be exposed to the compound of interest in step i) for a period of between about 5 minutes and about 24 hours. For example, the protein target may be exposed to the compound of interest in step i) for a period of between about 0.5 hours and about 4 hours. In a suitable embodiment, the protein target is exposed to the compound of interest in step i) for a period of between about 1 hours and about 2 hours.

### Contacting proteins with a chemical denaturing agent

The methods of the invention require a protein target (in the methods of the first aspect of the invention) or an associated protein (in the methods of the second aspect) to be contacted with a chemical denaturing agent. This produces a denaturing mixture. Details of suitable chemical denaturing agents and the constituents of denaturing mixtures are considered elsewhere in the specification.

The following considerations regarding "proteins" apply to contacting protein targets (in methods of the first aspect of the invention or second aspect) and/or associated proteins (in methods of the second aspect) with chemical denaturing mixtures, unless the context requires otherwise.

The extent of potential denaturation that may occur can be controlled by varying the period of time for which the protein and chemical denaturing agent are contacted with one another and/or varying the denaturant concentration.

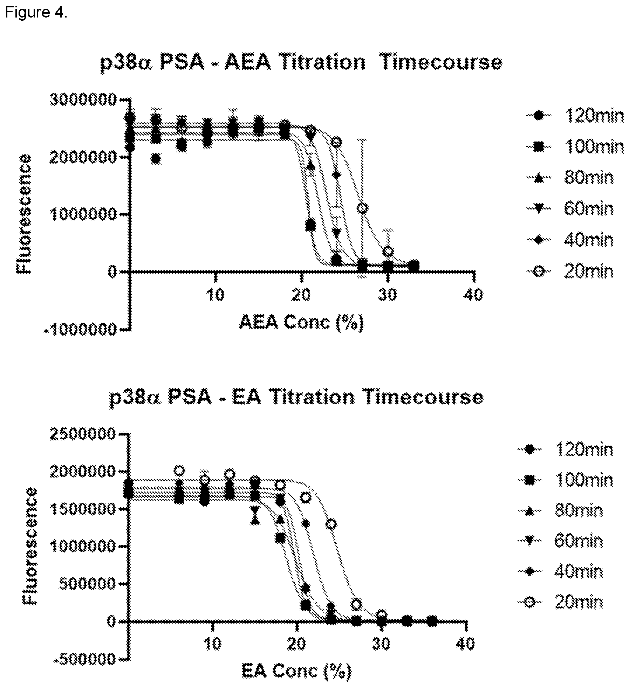

Suitably, the protein may be contacted with the chemical denaturing agent for a period of between approximately 5 minutes and approximately 8 hours. By way of example, the protein may be contacted with the chemical denaturing agent for a period of between approximately 1 hours and approximately 3 hours. In a suitable embodiment, the protein may be contacted with the chemical denaturing agent for a period of between approximately 30 minutes and approximately 24 hours. Suitably, the protein may be contacted with the chemical denaturing agent for a period of approximately 2 hours. Optimal time of contact with the chemical denaturing mixture (i.e. the time at which assay completion has been reached) can be determined by the running of a time course experiment where assays in the form of titration curves for the chemical denaturing agent are started sequential by the addition of lysate to each assay at an appropriate time and stopped simultaneously by the addition of detection reagent (e.g. see Figure 4).

Step ii) of a method of the invention may involve contacting the protein with the chemical denaturing agent for a period of up to four hours. Step ii) of a method of the invention may involve contacting the protein with the chemical denaturing agent for a period of up to three hours. Suitably, step ii) of a method of the invention involves contacting the protein with the chemical denaturing agent for a period of up to two hours.

Alternatively, or additionally, the extent of potential denaturation that may occur can be controlled by varying the concentration of the denaturing agent with which the protein target is contacted. In the assay, a set volume of denaturant is added to the cell lysate or intact cell suspension to generate the desired final concentration of denaturant that the target protein is exposed to. In the event that a denaturant concentration-response experiment is planned, a range of denaturant 'stock solutions' are prepared at different concentrations and a set volume of each is then transferred to the cell lysate. For example, this may be achieved with a 1:2, or 1:5, or 1:10 dilution.

In an embodiment of the invention in which the protein target is provided in the form of a cell lysate, the final concentration of the cell lysate in a denaturing mixture may be between about 0.1 ng/µl and 5 µg/µl. Suitably, the final concentration of the cell lysate in a denaturing mixture may be between about 1ng/ul and 2.0 µg/µl. For example, the final concentration of the cell lysate in a denaturing mixture may be between about 0.4 µg/µl and 0.8 µg/µl.

In an embodiment of the invention in which the protein target is provided in the form of intact cells, the final density of cells in a denaturing mixture may be between about 10,000 and 100,000 per well. Suitably, the final density of cells in a denaturing mixture may be between about 20,000 and 75,000 cells per well. For example, the final density of cells in a denaturing mixture may be between about 40,000 and 60,000 cells per well.

Suitably, in the case of a method of the invention practiced in a 384 well plate, or other multiwell plate, the pre-determined number of cells may be at least, for example between about 10,000 and 100,000 cells per well of a 384-well plate, such as approximately 50,000 cells per well, The skilled person will appreciate that these numbers may be varied, *mutatis mutandis,* in the case that a vessel of a different size is used.

Concentrations that may be used in respect of certain specific denaturing agents are considered elsewhere in the specification, in connection with discussions of the relevant agents.

### A chemical denaturing agent

The methods of the invention make use of a chemical denaturing agent to bring about denaturation of the protein (which may be a protein target in a method of the first aspect of the invention, or an associated protein in a method of the second aspect). The denaturation does not require heat, and suitably denaturing is achieved without heating a mixture comprising the protein.

In principle, any chemical or mixture of chemicals capable of bringing about a requisite degree of denaturation of the protein may be used as a chemical denaturing agent in the methods of the invention. Merely by way of example, one or more of: a solvent; and/or a detergent; and/or a chaotropic agent may be employed as a suitable chemical denaturing agent. In the case where more than one such agent is to be used, the agents may be used in combination, with the mixture constituting the requisite chemical denaturing agent.

In the case that a solvent is to be used, one or more short chain organic solvents may be employed as a chemical denaturing agent in the context of the present invention.

As illustrated in the Examples, the inventors have found that a mixture comprising one or more of: an acid; and/or an alcohol; and/or a ketone, may constitute a suitable chemical denaturing agent for use in the methods of the invention. In a suitable embodiment, the chemical denaturing agent may comprise an acid (particularly a weak acid). In a suitable embodiment, the chemical denaturing agent may comprise an alcohol. In a suitable embodiment, the chemical denaturing agent may comprise a ketone. In a suitable embodiment, the chemical denaturing agent comprises an acid and an alcohol. In a suitable embodiment, the chemical denaturing agent comprises an acid and a ketone. In a suitable embodiment, the chemical denaturing agent comprises an alcohol and a ketone. The chemical denaturing agent may comprise each of an alcohol, a ketone and an acid (particularly a weak acid).

In the case of a chemical denaturing agent comprising a mixture of each of an acid, an alcohol, and a ketone, the alcohol and ketone may be provided in approximately equal quantities. The acid may be provided in a markedly smaller amount than either the alcohol or ketone. The alcohol may be provided in an amount greater than the ketone. Suitably, the alcohol is present in a mixture at a concentration of between about 30% and 100%. Suitably the alcohol content is at least 50%.

A ketone may be absent from the mixture, however, if present suitably the ketone is present in a mixture at a concentration of between about 0.1% and 70%. An acid may be absent from the mixture, however, if present suitably the acid is present in a mixture at a concentration of between about 0.01% and 0.5%.

Suitably, if the mixture comprises an alcohol and a ketone these may be present in a 50:50 ratio.

Suitably, if the mixture comprises an alcohol and a ketone there is a greater percentage of alcohol present than ketone.

The inventors have found that the methods of the invention may be practiced using a chemical denaturing agent that comprises an acid and an alcohol at differing ratios. A suitable ratio being of approximately 0.1:100. A ratio of 0.1 acetic acid:100 ethanol is particularly useful. The inventors have found that the methods of the invention may be practiced using a chemical denaturing agent that comprises an acid, an alcohol and a ketone at differing ratios. A suitable ratio being of approximately 0.1:50:50. A ratio of 0.1 acetic acid:50 ethanol: 50 acetone is particularly useful.

The inventors have found that the methods of the invention may be practiced using a chemical denaturing agent that comprise different examples of an alcohol, and a ketone and/or an acid.

### Acids

In the case where the chemical denaturing agent comprises an acid (such as in the case of a mixture comprising an acid), the chemical denaturing agent may comprise a carboxylic acid. Suitably, the mixture may comprise a 1-5C acid, such as a 1-5C carboxylic acid. Suitably, the chemical denaturing agent may be a mixture comprising a carboxylic acid selected from the group consisting of: acetic acid, formic acid, propanoic acid, butanoic acid, pentanoic acid. Suitably, the chemical denaturing agent may be a mixture comprising acetic acid.

In the case where the chemical denaturing agent is a mixture comprising an acid, the acid may be a carboxylic acid. Suitably the acid may be a 1-5C acid, such as a 1-5C carboxylic acid. Suitably, the acid may be a carboxylic acid selected from the group consisting of: acetic acid, formic acid, propanoic acid, butanoic acid, pentanoic acid.

The chemical denaturing agent may comprise a mixture comprising more than one acid.

### Alcohols

In the case where the chemical denaturing agent is a mixture comprising an alcohol, the mixture may comprise a 2-5C alcohol. Suitably, the chemical denaturing agent may be a mixture comprising an alcohol selected from the group consisting of: ethanol; propanol; butanol; and pentanol. In the case that propanol is used, the propanol may be propan-1-ol. Suitably, the chemical denaturing agent may be a mixture comprising ethanol or propanol. Suitably the chemical denaturing agent may be a mixture comprising ethanol.

In the case where the chemical denaturing agent is a mixture comprising an alcohol, the alcohol may be a 2-5C alcohol. Suitably, the alcohol may be selected from the group consisting of: ethanol; propanol; butanol; and pentanol.

The chemical denaturing agent may comprise a mixture comprising more than one alcohol.

### Ketones

In the case where the chemical denaturing agent is a mixture comprising a ketone, the mixture may comprise a 2-5C ketone. Suitably, the chemical denaturing agent may be a mixture comprising a ketone selected from the group consisting of: acetone; propanone; butanone; and pentanone. Suitably, the chemical denaturing agent may be a mixture comprising acetone or butanone. In the case that butanone is used, the butanone may be 2-butanone.

In the case where the chemical denaturing agent is a mixture comprising a ketone, the ketone may be a 2-5C ketone. Suitably, the ketone may be selected from the group consisting of: acetone; propanone; butanone; and pentanone.

The chemical denaturing agent may comprise a mixture comprising more than one ketone.

### Mixtures

The inventors have demonstrated that the assay can be operated using a wide range of denaturing agents.

Suitably, a method of the invention may employ a chemical denaturing agent selected from the group consisting:
- a mixture comprising: acetic acid; and ethanol; and acetone;
- a mixture comprising: acetic acid; and ethanol; and butanone;
- a mixture comprising: acetic acid; and propanol; and butanone;
- a mixture comprising: acetic acid; and ethanol;
- a mixture comprising: acetic acid; and propanol;
- a mixture comprising: formic acid; and ethanol; and acetone;
- a mixture comprising: formic acid; and ethanol; and butanone;
- a mixture comprising: formic acid; and propanol; and butanone;
- a mixture comprising: formic acid; and ethanol;
- a mixture comprising: formic acid; and propanol;
- a mixture comprising: propanoic acid; and ethanol; and acetone;
- a mixture comprising: propanoic acid; and ethanol; and butanone;
- a mixture comprising: propanoic acid; and propanol; and butanone;
- a mixture comprising: propanoic acid; and ethanol;
- a mixture comprising: propanoic acid; and propanol;
- a mixture comprising: butanoic acid; and ethanol; and acetone;
- a mixture comprising: butanoic acid; and ethanol; and butanone;
- a mixture comprising: butanoic acid; and propanol; and butanone;
- a mixture comprising: butanoic acid; and ethanol;
- a mixture comprising: butanoic acid; and propanol;
- a mixture comprising: pentanoic acid; and ethanol; and acetone;
- a mixture comprising: pentanoic acid; and ethanol; and butanone;
- a mixture comprising: pentanoic acid; and propanol; and butanone;
- a mixture comprising: pentanoic acid; and ethanol;
- a mixture comprising: pentanoic acid; and propanol;
- a mixture comprising: ethanol; and acetone;
- a mixture comprising: ethanol; and butanone;
- a mixture comprising: propanol; and acetone;
- a mixture comprising: propanol; and butanone;
- a denaturant solution comprising; ethanol alone; and
- a denaturant solution comprising; propanol alone.

Suitably, a method of the invention may employ a chemical denaturing agent selected from the group consisting of:
- a mixture comprising: acetic acid; and ethanol; and acetone (optionally in a ratio of 0.1:50:50);
- a mixture comprising: acetic acid; and ethanol; and butanone (optionally in a ratio of 0.1:50:50);
- a mixture comprising: acetic acid; and propanol; and butanone (optionally in a ratio of 0.1:50:50).
- a mixture comprising: acetic acid; and ethanol (optionally in a ratio of 0.1:100);
- a mixture comprising: acetic acid; and propanol (optionally in a ratio of 0.1:100);
- a mixture comprising: formic acid; and ethanol; and acetone (optionally in a ratio of 0.1:50:50);
- a mixture comprising: formic acid; and ethanol; and butanone (optionally in a ratio of 0.1:50:50);
- a mixture comprising: formic acid; and propanol; and butanone (optionally in a ratio of 0.1:50:50).
- a mixture comprising: formic acid; and ethanol (optionally in a ratio of 0.1:100);
- a mixture comprising: formic acid; and propanol (optionally in a ratio of 0.1:100);
- a mixture comprising: propanoic acid; and ethanol; and acetone (optionally in a ratio of 0.1:50:50);
- a mixture comprising: propanoic acid; and ethanol; and butanone (optionally in a ratio of 0.1:50:50);
- a mixture comprising: propanoic acid; and propanol; and butanone (optionally in a ratio of 0.1:50:50).
- a mixture comprising: propanoic acid; and ethanol (optionally in a ratio of 0.1:100);
- a mixture comprising: propanoic acid; and propanol (optionally in a ratio of 0.1:100);
- a mixture comprising: butanoic acid; and ethanol; and acetone (optionally in a ratio of 0.1:50:50);
- a mixture comprising: butanoic acid; and ethanol; and butanone (optionally in a ratio of 0.1:50:50);
- a mixture comprising: butanoic acid; and propanol; and butanone (optionally in a ratio of 0.1:50:50).
- a mixture comprising: butanoic acid; and ethanol (optionally in a ratio of 0.1:100);
- a mixture comprising: butanoic acid; and propanol (optionally in a ratio of 0.1:100);
- a mixture comprising: pentanoic acid; and ethanol; and acetone (optionally in a ratio of 0.1:50:50);
- a mixture comprising: pentanoic acid; and ethanol; and butanone (optionally in a ratio of 0.1:50:50);
- a mixture comprising: pentanoic acid; and propanol; and butanone (optionally in a ratio of 0.1:50:50).
- a mixture comprising: pentanoic acid; and ethanol (optionally in a ratio of 0.1:100);
- a mixture comprising: pentanoic acid; and propanol (optionally in a ratio of 0.1:100);
- a mixture comprising: ethanol; and acetone (optionally in a ratio of 50:50);
- a mixture comprising: ethanol; and butanone (optionally in a ratio of 50:50);
- a mixture comprising: propanol; and acetone (optionally in a ratio of 50:50);
- a mixture comprising: propanol; and butanone (optionally in a ratio of 50:50);
- a denaturant solution comprising; ethanol alone; and
- a denaturant solution comprising; propanol alone.

Whilst the denaturing agents comprising a mixture of a carboxylic acid, and/or an alcohol, and/or a ketone may be stored for a period between their manufacture and use it is preferred if the mixture has not been stored for any excessive length of time, and/or is prepared fresh prior to use. Accordingly, in a suitable embodiment, a chemical denaturing agent comprising a mixture of at least two of: an acid; an alcohol; and a ketone is produced no more than 1 week prior to use to denature a protein target (for example in a method of the invention). Suitably, such a mixture is produced between 0 and 24 hours prior to use. For example, a mixture may suitably be produced directly prior to use.

In a suitable embodiment, a chemical denaturing agent comprising a mixture of an alcohol and a ketone is produced no more than 1 week prior to use to denature a protein target (for example in a method of the invention). Suitably, such a mixture is produced between 0 and 24 hours prior to use. For example, a mixture may suitably be produced directly prior to use.

If multiple assays are to be performed over various days or weeks, for example as part of one overall program of work or experiment, it may be desirable to prepare a large batch of the mixture sufficient for use in all these assays.

### Distinguishing reversible and irreversible binders

By comparing a reversible and irreversible inhibitor of p38a MAPK in a protein stability assay run at a suitable range of denaturant concentrations and concentration responses of compound, the inventors have recognised that the protein stability assay (PSA) of the present invention can be used to distinguish compound mechanism-of-action; e.g. determine whether a compound is a reversible or irreversible binder to its target protein (Figure 9, Example 6).

Thus, according to particular embodiments of the invention the method of the first aspect of the invention or second aspect can distinguish whether the compound binds to the protein in a reversible or irreversible manner.

In particular embodiments, the test compound is identified as being a reversible inhibitor or an irreversible inhibitor based on the EC₅₀ values across a range of denaturant concentrations and/or the shape of the curves across range of denaturant concentrations. Example of this can be seen in Figure 9.

The EC₅₀ values across a range of denaturant concentrations (typically within a 4% or 5% range; e.g. 18%-22% is a 5% range) and/or the shape of the curves at percent points across range of denaturant concentrations (e.g. 18%, 19%, 20%, 21%, 22%) can be used to identify whether the test compound is a reversible or irreversible inhibitor.

Suitably, the test compound can be identified as being a reversible inhibitor if it yields or demonstrates a 10-fold or greater change in EC₅₀ values across a 5% denaturant concentration range. Additionally, the curve shapes differ at different denaturant concentrations; whereas an irreversible inhibitor demonstrates more consistent EC₅₀ values (typically within a 4-fold range) within a 5% denaturant concentration range. Additionally, the curve shape remains relatively consistent.

In a particular embodiment, the test compound can be identified as being a reversible inhibitor if it demonstrates a 10-fold or greater change in EC₅₀ values across a 5% denaturant concentration range. In particular embodiments, the test compound can be identified as being a reversible inhibitor if it demonstrates 12-fold, 15-fold, 20-fold, 25-fold or greater change in EC₅₀ values across a 5% denaturant concentration range.

In a particular embodiment, the test compound can be identified as being an irreversible inhibitor if it demonstrates EC₅₀ values across a 5% denaturant concentration range that are within 4-fold of each other across the range. In a particular embodiments, the test compound can be identified as being an irreversible inhibitor if it demonstrates EC₅₀ values across a 5% denaturant concentration range that are within 4-fold, 3-fold or 2-fold of each other across the range.

In a particular embodiment, the test compound can be identified as being a reversible inhibitor if it demonstrates a 10-fold or greater change in EC₅₀ values across a 4% denaturant concentration range. In particular embodiments, the test compound can be identified as being a reversible inhibitor if it demonstrates 12-fold, 15-fold, 20-fold, 25-fold or greater change in EC₅₀ values across a 4% denaturant concentration range.

In a particular embodiment, the test compound can be identified as being an irreversible inhibitor if it demonstrates EC₅₀ values across a 4% denaturant concentration range that are within 4-fold of each other across the range. In a particular embodiments, the test compound can be identified as being an irreversible inhibitor if it demonstrates EC₅₀ values across a 4% denaturant concentration range that are within 4-fold, 3-fold or 2-fold of each other across the range.

In a particular embodiment the method of the first aspect of the invention or second aspect is performed using a range of denaturant concentrations, such as a 3%, 4% or 5% range; and concentration response curves are generated and EC₅₀ values determined for each curve.

From a comparison of compound concentration response curves generated and the range of EC₅₀ values across the range of concentrations used, it is possible to distinguish between reversible and irreversible modes of action of the compound.

Suitably, the "range of denaturant concentrations" spans the denaturant concentrations where a compound-induced stabilisation shift is evident in the protein unfolding curve, as defined by denaturant titration experiments (e.g. if a stabilisation curve shift of target protein occurs between 19 and 20% ethanol/acetic acid (EA), compound concentration response curves using denaturant concentrations of 18%, 19%, 20% and 21% EA would reflect a 4% range of denaturant concentrations). Based on the shape of the curve or the change in EC₅₀ values determined for each curve it is possible to ascertain whether the test compound is a reversible or irreversible inhibitor.

### Denaturing agent toolkit

The inventors have discovered that various denaturing agent solutions (such as pure alcohol, mixtures of alcohol or ketone, mixtures of alcohol and acid, or mixtures of alcohol, ketone and acid, in various ratios) can be used in the practice of the methods of the invention. However, certain denaturing agents may be more appropriate for certain target proteins or test compounds.

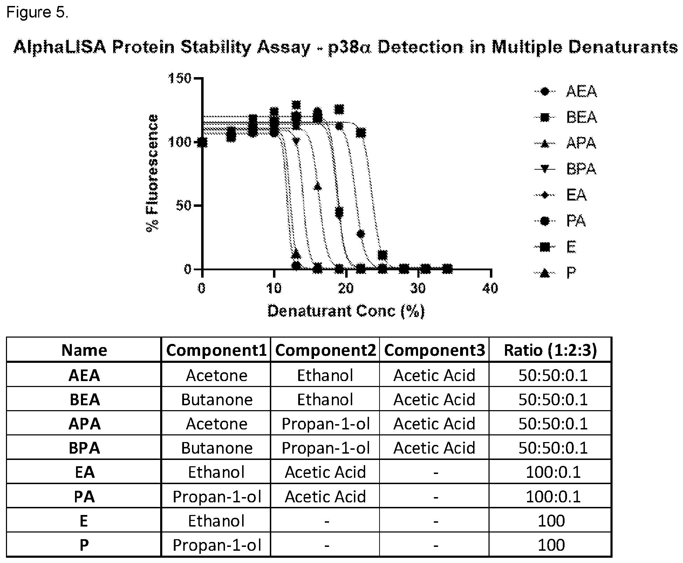

Accordingly, the methods of the invention can be practiced with a range of denaturant agent in parallel so as to determine a suitable denaturing agent for use when testing the stability of a particular target protein (Figure 5). The ability to conduct the method in a multiwell plate, such as a 96-, 384- or 1536-well plate allows such parallel testing.

An example of the toolkit approach, where a range of chemical denaturants are utilised in the protein stability assay (e.g. Acetone/Ethanol/Acetic Acid (AEA), ethanol/acetic acid (EA), ethanol (E), butanone/propanol/acetic acid (BPA), propanol/acetic acid (PA), propanol (P)) is described Example 3 and Figure 5.

As can be seen from Figure 5, there are differences between these chemical denaturants - e.g. they unfold the target protein at different concentrations. This may be advantageous, for example if a high concentration interferes with the assay readout.

Thus, utilising this toolkit approach facilitates the identification of a suitable chemical denaturant to use in any particular assays system (e.g. with a particular target protein).

Suitably, the methods of the first aspect of the invention or second aspect can be employed using a plurality of distinct formulations of denaturing agent. Suitably, each of the distinct formulations of denaturing agent is tested in parallel. Suitably, each of the distinct formulations of denaturing agent is tested in parallel with the same protein target and a standard or reference test compound. Suitably, each of the distinct formulations of denaturing agent is tested in a separate well of a multiwell plate. By testing the various formulations of denaturing agent alongside one another under the same circumstances with a particular target protein the operator is able to select a suitable, e.g. a preferred, denaturation agent formulation for subsequent use with that target protein.

Thus, by way of example a grid pattern can be set up to test different mixtures of compounds as denaturing agent (different formulation) and/or different ratios of each component.

For example, the test formulations could be an alcohol selected from: ethanol, propanol, pentanol and butanol; an acetone selected from acetone; propanone; butanone; and pentanone; an acid selected from the group consisting of: acetic acid, propanoic acid, butanoic acid and pentanoic acid. Furthermore, each component in the mixture can be present in differing ratios, e.g. 10%, 20%, 30%, 40%, 50%, 60%, 70%, 80%, 90% 100% of one and the remaining percentage to 100% of one or more agents (e.g. see Figure 5).

In this way a toolkit plate assay is employed to select a denaturing agent formulation for further use in testing compounds for their ability to interact with/bind to a target protein. Use of such a toolkit plate would allow an appropriate denaturing agent to be selected, that over a given concentration range would not adversely interfere with the assay endpoint. Such a methodology is important to create an assay capable of the assessment of a range of protein targets with varying stabilities. The toolkit plate assay enables proteins usually requiring a denaturant concentration range beyond the limits of the assay endpoint to still be assessed using the protein stability assay. In addition, different denaturants may be optimal for studying a specific target protein or target protein:compound combination.

### A denaturing mixture

A denaturing mixture in the context of the present invention comprises the protein (a protein target in a method of the first aspect of the invention, or an associated protein in a method of the second aspect) and the chemical denaturing agent. A denaturing mixture may optionally further comprise the compound of interest. The compound of interest within a denaturing mixture may be bound to the protein target, or may be unbound.

A protein in a denaturing mixture may be present in its folded state, or in its denatured state. A protein in its denatured state may be soluble ("unfolded"), or insoluble ("precipitated"). A protein in a denaturing mixture may be provided in a purified form or may be present in a mixture of proteins, e.g. in a cell lysate, or may be present in cells; thus, the denaturing mixture may comprise whole cells or a cell lysate that comprises the protein of interest.

### Products of a denaturing mixture

Step iii) of the methods of the invention involves assaying the products of the denaturing mixture. The purpose of this step is to investigate the protein constituents of the denaturing mixture to determine the proportion of the protein target or associated protein that is present in its folded state, as compared to the proportion of the protein target or associated protein that is present in its denatured state.

The products of the denaturing mixture are the constituents present after the denaturing agent and the protein target or associated protein have been in contact for sufficient time to bring about a denaturing of the relevant protein. The assay can be set up such that denaturation reaches a stable point of equilibrium between denatured and native protein, however the assay can also be conducted with shorter incubation periods. The methods of the invention can also be carried out using time-course measurements, e.g.
step (ii) can be carried out for differing periods (e.g. 5, 10, 15, 20, 30, 60, 80, 100, 120 minutes). In this way, the operator may find that the data shifts with increased exposure time to the denaturing agent, in relation to the amount of protein denaturing that occurs.

The products may be those present once the denaturation process occurring in step ii) of a method of the invention has reached equilibrium or at an earlier stage. It will be recognised that the extent to which denaturation has, or has not, occurred will depend upon a number of factors, including resistance of the relevant protein to denaturation, the strength of the chemical denaturing agent and time for which it is in contact with the protein, as well as any impact of the compound of interest on the stability of the protein in question.

As discussed further elsewhere, the products of the denaturing mixture may be assayed at a point where they are still present in the denaturing mixture, or may be assayed at a point where they have been separated from one or more other constituents of the denaturing mixture. Accordingly, the products of the denaturing mixture may be assayed in the presence of the chemical denaturing agent, or in the absence of the chemical denaturing agent. Similarly, the products of the denaturing mixture may be assayed in the presence of the compound of interest, or in the absence of the compound of interest.

In particular embodiments, the methods of the first aspect of the invention or second aspect can be employed without the need for separation of one or more constituents from others in the denaturing mixture and so method steps ii) and iii) or i), ii) and iii) are carried out in the same vessel. In particular embodiments, method steps ii) and iii) or i), ii) and iii) are carried out without a rigorous centrifugation.

In the event that the products of the denaturing mixture are to be separated from one or more other constituents of the denaturing mixture, this may be practiced by any suitable mechanism known to those skilled in the art. Merely by way of example, a denaturing mixture may be subject to centrifugation or filtration, and the products of the denaturing mixture separated in this manner.

### Folded and denatured states

For the purposes of the present invention, references to a protein target or associated protein in a denatured state may be taken as encompassing molecules of the protein that have lost some or all of their quaternary, tertiary or secondary structures. The skilled person will be well aware of means and methods by which the presence or absence of such structures may be assessed. Illustrative examples of these are considered elsewhere in the present disclosure.

In a suitable embodiment, a protein in a denatured state is unfolded, but remains soluble so that it is not precipitated. The skilled person will be readily able to select assays that may be used to assess the proportion of a protein that is present in an unfolded state. Suitably, such assays may not require the folded and soluble unfolded protein to be separated from one another. Examples of such assays are described further below.

In a suitable embodiment, a protein in a denatured state is precipitated. The skilled person will be readily able to select assays that may be used to assess the proportion of a protein that is present in a precipitated state. Examples of such assays are Western blot and Mass Spectrometry.

Precipitated protein may be readily separated from unprecipitated protein by methods well known to those skilled in the art, such as filtration or centrifugation, though suitable assays may not necessarily require the precipitated protein and folded protein to be separated from one another.

### Assaying

The assaying performed in step iii) of a method of the invention is performed in respect of a protein target (in the methods of the first aspect of the invention) or an associated protein (in the methods of the second aspect). For the sake of simplicity, references to "proteins" in this part of the disclosure should be considered to encompass either protein targets or associated proteins, as required.

The assaying step determines the proportion of the protein present in its folded state and/or in its denatured state. As explained elsewhere, since all protein present will be in either folded or denatured state, if the total quantity of protein present is known, then knowledge of the amount of folded protein present can allow the amount of denatured protein present to be determined, and knowledge of the amount of denatured state present can allow the amount of folded protein present to be determined.

A suitable assaying step will be able to distinguish between the protein in its folded state and the protein in its denatured state. In order to achieve this, the assaying used is able to detect the protein in its folded state and/or the protein in its denatured state.

Typically, the proportion of the protein target in its folded or unfolded state will be determined by detection of the protein target itself. In particular embodiments, in step (iii) of the methods of the invention, direct measurement of the protein target is used to determine the proportion of the protein target in its folded state and/or the proportion of protein in its denatured state. In certain circumstances it may be possible to determine the proportion of the protein target in its folded or unfolded state indirectly by detection of an associated protein. In particular embodiments, in step (iii) measurement of an associated protein is used to determine the proportion of the protein target in its folded state and/or the proportion of protein in its denatured state.

Suitable assays for use in step iii) are capable of distinguishing between different conformations of the protein. In particular, they will be able to distinguish between the protein in its folded state, and in its denatured state. As set out above, a denatured protein may be unfolded, but remain soluble, or may be precipitated.

In a suitable embodiment, the assaying of step iii) detects the protein in its folded state. Suitably, the assaying used may be able to quantify the amount of the protein present in its folded state. In a suitable embodiment, the assaying of step iii) detects the protein in its denatured state. Suitably, the assaying used may suitably be able to quantify the amount of the protein present in its denatured state.

### Assaying the products of a denaturing mixture

Step iii) of the methods of the invention involves assaying the products of the denaturing mixture. The term "products of a denaturing mixture" is discussed in more detail above.

As set out above, the products of the denaturing mixture may be assayed at a point where they have been separated from one or more other constituents of the denaturing mixture, or may be assayed at a point where they are still present in the denaturing mixture.

In embodiments of the methods of the invention in accordance with this latter option, the assaying referred to in step iii) may be carried out directly upon the denaturing mixture produced in step ii). By "directly", it is meant that there is no separation of the constituents of the denaturing mixture, e.g. using a rigorous centrifugation step, prior to the assay being conducted. Previous approaches to investigating target engagement via changes in protein stability have frequently involved the separation of soluble and insoluble components prior to analysis. This is not required in these embodiments of the methods of the present invention.

Thus, in particular embodiments the methods of the invention comprise: step iii) directly assaying the products of the denaturing mixture to determine the proportion of the protein target (for first aspect of the invention) or associated protein (for second aspect) in its folded state and/or the proportion of the protein target (for first aspect of the invention) or associated protein (for second aspect) in its denatured state.

In the event that the products of the denaturing mixture are to be assayed as part of the denaturing mixture, then this may be practiced by assaying the denaturing mixture *in situ* in the vessel in which it was produced. Embodiments in accordance with this option offer advantages in that they reduce the need for transfers between different vessels, again increasing simplicity of the methods, and lending themselves to their use in high throughput applications.

As discussed elsewhere, methods of the invention in accordance with this embodiment are well suited to use in high throughput applications, in that the ability to exclude a separation step, which in practice invariably requires transfer of constituents to a new vessel, simplifies automation of the methods.

However, the methods of the invention can be practiced using an assaying step carried out on products of the denaturing mixture that have been separated from one or more other constituents of the denaturing mixture. This approach may be favoured in respect of certain forms of assay, such as Western blotting or mass spectroscopy discussed below. It will be appreciated that separation may be facilitated by utilising methods in which the protein to be assayed is precipitated. In such cases, step ii) of the method of the invention may employ conditions that favour the precipitation of denatured protein. For example, such methods may use an extended period of denaturation during step ii), or a higher concentration of the chemical denaturing agent.

### Exemplary assays for determining the states of proteins in the products of denaturing mixtures

The skilled person will be aware of many suitable assays by which the state of proteins in the products of denaturing mixtures can be determined. With that in mind, the examples provided below should be taken as non-limiting and for illustration only.

Suitably, an assay for determining the state of products of denaturing mixtures may be based upon the ability to detect changes in the "length" of a protein that occur as the protein is denatured and unfolds. Examples of such assays may employ at least two affinity reagents (such as a pair of antibodies) that bind to different sites on the protein. The affinity reagents may be labelled in such a manner that a signal is only generated when the two binding agents are close enough in proximity to one another (when the protein is in folded form), and no signal is generated when the two binding agents are relatively further away from one another when the protein is in denatured (and hence at least partially unfolded) form. The amount of the signal present thus provides an indication of the amount of folded protein present. Alternatively, loss of signal in an assay of this format may reflect a loss of accessibility of one or both of the sites where the antibodies bind.

Merely by way of example, reagents suitable for use in such assays are commercially available under the name "AlphaLISA^{®}".

HTRF (Homogeneous Time Resolved Fluorescence) is a no-wash technology with improved signal to noise ratio. It combines standard FRET technology with time-resolved measurement of fluorescence, eliminating short-lived background fluorescence. Two antibodies that recognize the target protein are used, with one antibody coupled to a donor, and the other with the acceptor. When the two antibodies bind to the protein in close enough proximity (when the protein is in its folded form), the donor will emit fluorescence upon excitation and the energy will be transferred to the nearby acceptor, giving specific acceptor fluorescence. The donor fluorescence is also measured and a ratio with the acceptor fluorescence is applied.

Merely by way of example, reagents suitable for use in such assays are commercially available under the name "HTRF^{®} (Homogeneous Time Resolved Fluorescence)".

Suitably, an assay for determining the state of products of denaturing mixtures may be based upon the ability to detect changes in the accessibility of a peptide tag fused to the protein that occur as the protein is denatured and unfolds. Examples of such assays may employ a tag, which may be an enzyme fragment and may be a fusion protein with a protein macromolecule. After contact with the denaturing mixture, the proportion of accessible tag is determined by addition of a complementary enzyme fragment which will complement the tag on macromolecules in which denaturation was minimized by the binding of the test compound. The addition of complementary enzyme fragment mixture also produces a dilution of the denaturant mixture, to prevent denaturation of the enzyme fragment. An enzyme substrate or substrate mixture is added to the active enzyme found in the mixture, and a luminescent reaction is read using standard optical methods to determine the proportion of folded protein remaining in the mixture.

Merely by way of example, reagents suitable for use in such assays are commercially available under the name "HiBiT^{®}" and "Nano-Glo^{®} HiBiT^{®} Lytic Detection System".

HiBit^{®} is suited for use where high throughput is required and/or particularly suited where no suitable target antibodies exist.

In particular embodiment, the methods of the first aspect of the invention or second aspect are carried out using an assay in step (iii) selected from the group consisting of: Homogeneous proximity assay (e.g. AlphaLISA^{®} or HTRF), Enzyme fragment complementation assay (e.g. HiBiT^{®}/Nanoluciferase), Western blot and Mass Spectrometry.

Methods selected from homogeneous proximity assays or enzyme fragment complementation assays are suitable for use in high-throughput formats. Such methods re well know. For example, Homogeneous proximity assay (e.g. AlphaLISA^{®} and HTRF) are disclosed in:
AlphaLISA^{®} - Beaudet, L., Rodriguez-Suarez, R., Venne, MH. et al. AlphaLISA immunoassays: the no-wash alternative to ELISAs for research and drug discovery. Nat Methods 5, an8-an9 (2008)
HTRF - Mathis, G. Probing molecular interactions with homogeneous techniques based on rare earth cryptates and fluorescence energy transfer. Clinical chemistry, 41(9), pp.1391-1397 (1995)

Enzyme fragment complementation assay (e.g. HiBiT^{®}/Nanoluciferase) are disclosed in:
HiBiT^{®} - Schwinn, M. K. et al. CRISPR-Mediated Tagging of Endogenous Proteins with a Luminescent Peptide. ACS Chem Biol 13, 467-474, https://doi.org/10.1021/acschembio.7b00549 (2018).
Nanoluciferase - Dixon, A. S., Schwinn, M. K., Hall, M. P., Zimmerman, K., Otto, P., Lubben, T. H., Butler, B. L., Binkowski, B. F., Machleidt, T., Kirkland, T. A., Wood, M. G., Eggers, C. T., Encell, L. P., and Wood,K. V. (2016) NanoLuc Complementation Reporter Optimized for Accurate Measurement of Protein Interactions in Cells. ACS Chem. Biol. 11, 400-408.

The assays referred to above provide examples of assays able to distinguish between proteins in different states within an unseparated mixture. Such assays that are able to distinguish between different states of the same protein in an unseparated mixture have advantages when used in the methods of the invention, in that they reduce complexity by avoiding the need for a separation step.

Other suitable assays that may be used in step iii) of a method of the invention rely upon the separation of folded and denatured proteins found within the products of a denaturing mixture.

Assays of this sort need not be able to distinguish between the folded and denatured states of the protein, as the separation step carried out means that only one state (folded or denatured) should be present in a sample to be investigated.

The skilled person will appreciate that assays that can be used in determining the state of proteins in products of denaturing mixtures in non-separated form will generally also be suitable for use in determining the state of such in separated form. The skilled person will be able to select appropriate means in order to assay the products of a denaturing mixture whether in separated or non-separated form. The skilled person will also be able to provide the products of a denaturing mixture in separated or non-separated form in order to meet the requirements of any chosen assaying means.

### Determining proportions of proteins in the folded or denatured states

As set out above, the assaying performed in step iii) of a method of the invention is able to distinguish between the folded state of a protein target and the protein target in its denatured state, whether separated or not. Suitably the assaying may be able to detect and quantify the amounts of the protein present in either of these states.

Since any given protein molecule will be in either a "folded state" or "denatured state" (i.e., these states are essentially binary - with any protein molecule being either one or the other) the skilled person will recognise that, if the total amount of a target protein present is known, it is only necessary to determine the amount that is in one of the two states, in order also to know the amount present in the other of the two states. Accordingly, a suitable assay may be selected to acquire information about any of the amounts referred to above, and from that information allow the proportions of folded and denatured protein target to be determined.

The relevant proportions may be calculated on the basis that any protein target present that is not folded is considered to be in a denatured state (as defined elsewhere herein) and that any protein target present that is not in a denatured state is considered to be in a folded state.

Thus, if the total amount of the target protein present in the products of a denaturing mixture is known, then the proportion of protein in its folded state and in its denatured state may be determined from any one (or more) of the following:
- knowledge of the amount of folded protein present; or
- knowledge of the amount of denatured protein present; or
- knowledge of the amounts of folded and denatured protein present.

### A change in the proportion of protein in its folded state and suitable controls

The methods of the invention make use of the changes in stability that occurs on binding of a compound of interest to a protein target to allow a determination to be made as to whether or not binding has taken place. As previously discussed, the change in stability may be an increase or decrease in stability, and may be observed in respect of the protein target itself, or another associated protein.

To establish that stability has changed, the proportion of the relevant protein (either protein target or associated protein) in its folded state (which as discussed above, is also indicative of the proportion of the protein in its denatured state) in compound treated samples is compared to a control samples not treated with compound or treated with vehicle only.

If the proportion of the protein in its folded state in compound treated samples is not different from that found in the untreated control samples, then this indicates that no change in stability has occurred, indicating that the compound of interest and protein target may not bind to one another.

If the proportion of the protein in its folded state in compound treated samples is greater than that found in the untreated control samples, then this indicates that the compound of interest and protein target do bind to one another, and that this binding causes an increase in stability of the protein target or associated protein (and hence an increase in the resistance of the protein to the denaturing conditions).

If the proportion of the protein in its folded state in the compound treated samples is lower than that found in the untreated control samples, then this also indicates that the compound of interest and protein target bind to one another, but in this case that the binding causes a decrease in stability of the protein target or associated protein (and hence an increase in the sensitivity of the protein to the denaturing conditions).

The proportion of protein in its folded state in the products of a denaturing mixture of a method of the invention may be compared with any appropriate comparator, with a view to determining whether or not an increase has occurred. The skilled person seeking to practice the invention will be well aware of suitable comparators that may be used, depending upon the purpose to which the method of the invention is being put.

Suitably, a comparator may be a sample of a mixture in which the amount of the compound of interest present is reduced or increased as compared to the denaturing mixture assayed. In a suitable example, a comparator may be a sample in which the compound of interest is not present. In such an example, the denaturing mixture may lack the compound of interest, but retain any carrier in which the compound of interest is otherwise provided. Merely by way of example, in the case where the compound of interest is provided in a DMSO carrier, a suitable control may comprise the same DMSO carrier, in the amount and concentration, but without the compound of interest.

Merely by way of example, a comparator may be a sample of a mixture in which the amount of the denaturing agent is reduced or increased as compared to the denaturing mixture assayed. Suitably, a comparator may be a sample in which the denaturing agent is not present.

Suitably, a comparator may be a sample of a mixture in which the amount of the protein target is reduced or increased as compared to the denaturing mixture assayed.

A suitable comparator may be part of, for example, a concentration curve prepared in respect of one or more of: the protein target; and/or the compound of interest; and/or the denaturing agent. Suitably the comparator may be part of a curve used for quantification.

In a suitable embodiment, a comparator may be a sample of a denaturing mixture assayed at a different timepoint. For example, the comparator may be a sample of the same denaturing mixture assayed at a different timepoint. In such an embodiment, the comparator may be a sample of the same mixture assayed at an earlier timepoint.

### Useful embodiments of the methods of the invention

The following passages provide details of embodiments of the methods of the invention that may be useful in particular applications. These suggestions are not limiting, and should be taken as illustrating the utility of the methods described herein.

### Methods suited to high throughput applications

High Throughput Screening (HTS) in drug discovery aims to identify active compounds from large compound libraries that modulate the activity of a biological target or pathway. Large numbers of known substances such as small molecule compounds can be rapidly screened in parallel against the biological target or pathway, rather than testing a small number of substances for activity across a wide range of targets (e.g. using high content analysis methods, such as proteomics). To achieve the level of throughput required, a relatively simple workflow incorporating assay designs amenable to automated sample, plate or liquid handling methods is important. Reproducible and robust data can be produced from efficient assay designs (e.g. a single test concentration in single-well assay performed in 96-, 384- or 1536-well formats). Homogenous assay approaches, which do not require sample transfer or wash steps are the most amenable to HTS applications.

As mentioned elsewhere in this document, certain embodiments of the methods of the invention lend themselves particularly well to use in high throughput applications. Accordingly, in the event that it is wished to use the methods of the invention in such a high throughput approach, then one or more (or indeed all) of the embodiments set out below may be employed.

A method of the invention intended to be used in a high throughput application may employ the same substantially constant temperature for steps ii) and iii) of the method. The method may employ the same substantially constant temperature for steps i) to iii) of the method. The substantially constant temperature may be ambient temperature.

Additionally, or alternatively, in a suitable embodiment of a method of the invention for high throughput use, steps ii) and iii) are conducted in the same vessel. Suitably, in such an embodiment each of steps i) to iii) are conducted in the same vessel. Suitably, the vessel in which the relevant steps are conducted may be a multiwell plate. Merely by way of example, a suitable multiwell plate may be a 96 well plate, a 384 well plate, a 1536 well plate, or the like.

Additionally, or alternatively, a method of the invention for high throughput use may involve assaying the products of the denaturing step directly in a denaturing mixture.

Additionally, or alternatively, a method of the invention to be used in high throughput applications employs an assay in step iii) that is able to distinguish the relevant protein in its folded and unfolded forms in an unseparated mixture. Examples of such assays have been described elsewhere in the specification. Such assays can thus be performed directly on the denaturing mixture produced in step ii).

The various embodiments described above having the common feature that they reduce or avoid the need to transfer constituents used in the methods from one container to another, thereby simplifying the methods of the invention, and increasing their suitability for use in high throughput applications.

Assays such as Homogeneous proximity assay (e.g. AlphaLISA^{®} or HTRF) and or enzyme fragment complementation assay (e.g. HiBiT^{®}/Nanoluciferase) are suitable for use in highthroughput formats.

Details of exemplary methods of the invention adapted for use in high throughput screening applications are set out in Examples 1 and 2 (using AlphaLISA^{®} testing compounds against p38α MAPK protein), Example 7 (using HiBiT^{®}), and Example 11 (reliable detection of active compound in a single-well per test compound screen).

In particular embodiments, the methods of the invention are used for screening at least 10, such as at least 50, at least 100, at least 150, at least 200, at least 500, at least 750, at least 1000, at least 1250, at least 1500, or more compounds at the same time. In practice the methods of the invention can be used to screen as many compounds as can be accommodated by the number of wells in a chosen multiwell plate.

### Methods using Western blotting

As described above, Western blotting represents a suitable method by which the assaying of step iii) of a method of the invention may be carried out. Western blotting constitutes an assay practiced in respect of products of a denaturing mixture that have been separated from one another.

Western blot analyses are usually carried out on the soluble fraction (measuring folded protein) but it can be done with either fraction. Prior to running the Western blot, it is usual to separate the folded and denatured proteins prior to analysis by centrifugation.

Western blot has a number of advantages, for example, if the operator wants to quickly evaluate a small number of compounds, it avoids the need to carry out a full assay development exercise. In addition, Western blot only requires a single antibody, not a pair of antibodies (cf, AlphaLISA^{®}, HTRF) and single antibodies are readily available for a high % of proteins.

Western blot also shows protein mass (band position) so gives added confidence in the antibody.

In a method of the invention in which assaying is to be performed using Western blotting, the products of the denaturing mixture suitably comprise denatured protein (whether a protein target or associated protein) in precipitated form. In keeping with this, the conditions used in step ii) of a method of the invention in which assaying is to be performed using Western blotting may be selected to favour the production of precipitated denatured protein.

Details of exemplary methods of the invention adapted for use in Western blot applications are set out in Example 5 of the Method Examples.

### Methods using mass spectroscopy

Mass spectroscopy represents another suitable method by which the assaying of step iii) of a method of the invention may be carried out using products of a denaturing mixture that have been separated from one another.

Since denatured and unfolded proteins are to be separated from one another, methods of the invention in which assaying is to be performed by mass spectroscopy may also make use of denatured proteins in either soluble or precipitated form. Accordingly, the conditions used in step ii) of a method of the invention in which assaying is to be performed using mass spectroscopy may be selected to favour the production of either soluble or precipitated denatured protein in the manner described elsewhere in the specification.

Mass Spectrometry (MS) does not require the use of antibodies (which may not exist for a target, or may not be specific) and critically MS allows the measurement of many proteins in parallel. The most frequently used MS method for CETSA measures ~5000 intracellular proteins in a single run. In general MS is slower and so better suited for deep profiling of a small number of compounds, than screening large compound numbers against a specified target.

The inventors have successfully utilised MS detection with the protein stability assay of the invention.

The following Examples and Figures serve to illustrate the invention. These Examples and Figures are in no way intended to limit the scope of the invention, but rather as examples from which equivalents will be recognized by those of ordinary skill in the art.

Unless it is apparent from the context, each of the embodiments listed above can be applied for use in any of the aspects of the invention.

### DESCRIPTION OF THE FIGURES

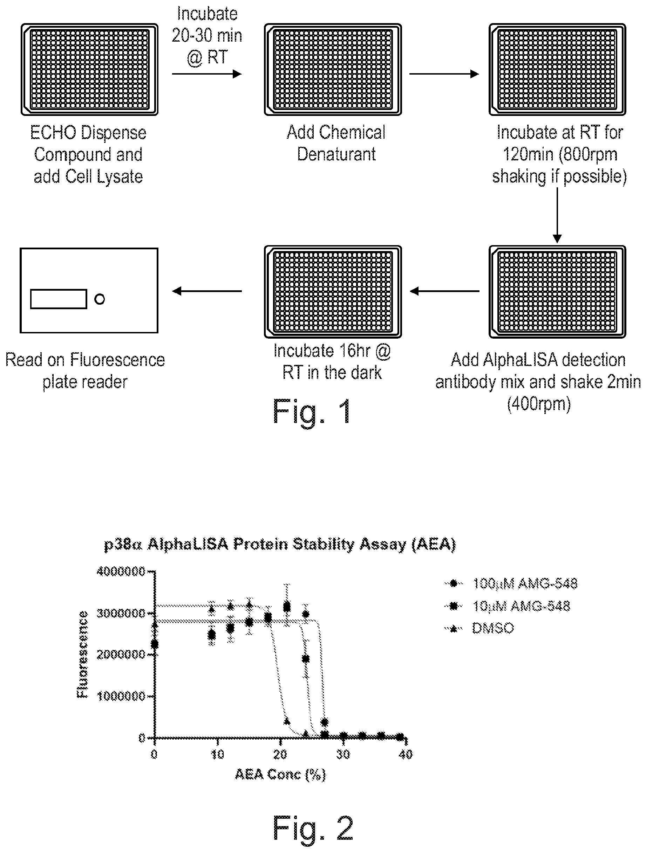

Figure 1: 'Protein Stability Assay' Schematic Using AlphaLISA^{®} Endpoint: Shows a cartoon schematic of Protein Stability assay workflow. The entire assay takes place in a single low volume microplate (e.g. 384-well) without the need for liquid transfers. Assay components are added sequentially to the plate, incubated at a consistent temperature (for example room temperature) as indicated and read using a fluorescent plate reader to allow relative target protein amounts to be detected. This workflow can be applied to assays using cell lysate or intact cells and a variety of endpoint technologies.
Figure 2: Demonstration of Protein Stabilisation in 'Protein Stability Assay' Using AEA as the Chemical Denaturant: Graph to show how the use of AEA (Acetone, Ethanol, Acetic Acid) as a denaturant can cause detectable protein unfolding in a p38α MAPK AlphaLISA^{®} endpoint assay. Soluble protein was measured by use of an in-house p38α MAPK AlphaLISA^{®} assay. Protein is unfolded by denaturant in a concentration dependent manner. Soluble protein is bound by the AlphaLISA^{®} reagents, while denatured protein is not. The addition of a fixed amount of AMG-548 compound (100 µM) as ligand can lead to stabilisation of protein in a dose dependent manner through the engagement of protein and ligand. This stabilisation results in a shift in curve shape as the amount of denaturant required to unfold the protein increases.

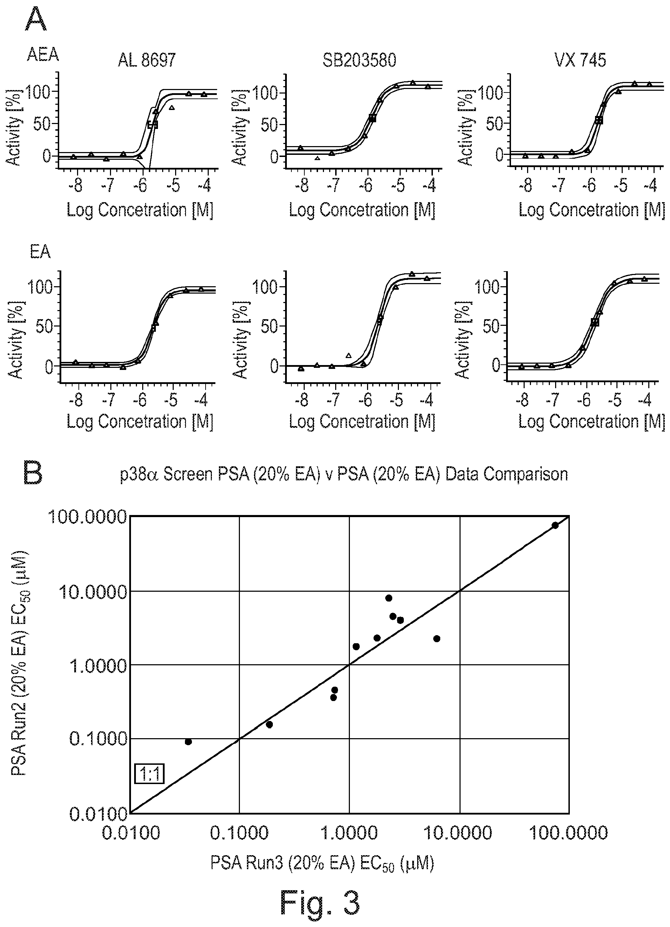

Figure 3A: Concentration response data for a panel of p38a inhibitors utilising the AlphaLISA^{®} protein stability assay with AEA or EA as the denaturant: Concentration response curves of three known p38a MAPK inhibitors (AL 8697, SB203580, VX 745) were generated using two different denaturant types. A set of commercially available compounds were screened in the p38a MAPK Protein Stability Assay using either AEA (Acetone, Ethanol, Acetic Acid) or EA (Ethanol, Acetic Acid) as the denaturant. The data generated demonstrates the stabilisation of protein in a dose dependent manner and shows how the use of different denaturants can create very similar profiles for each compound tested.
Figure 3b: Comparison of replicate EC₅₀ data between two screening runs of the Protein Stability Assay: Graph to show the reproducibility of data screened on different days in the same conditions. 18 compounds with a range of activities against target were screened in a p38a MAPK Protein Stability Assay on two separate occasions, using the same conditions. A 1:1 line has been plotted to indicate the agreement between the two sets of screening data. Run to run EC₅₀ values vary slightly, but ranking order remains the same.

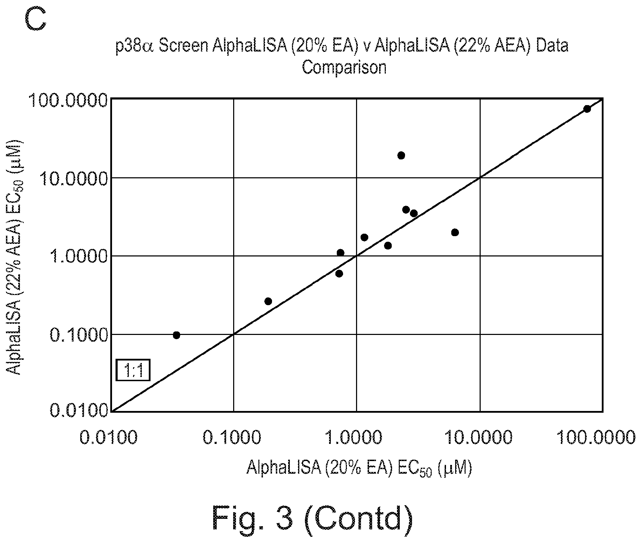

Figure 3c: Comparison of EC₅₀ data from the protein stability assay across two different denaturants: Graph to show the reproducibility of data screened on the same day in different denaturants. 18 compounds with a range of activities against target were screened in a p38a MAPK Protein Stability Assay on two separate occasions, using AEA (Acetone, Ethanol, Acetic Acid) or EA (Ethanol, Acetic Acid). A 1:1 line has been plotted to indicate the agreement between the two sets of screening data. Run to run EC₅₀ values vary slightly, but ranking order remains the same.
Figure 4: Denaturant time courses demonstrating completion of Protein Stability Assay to a stable endpoint: Graphs to show that if allowed to run, a point can be reached at which the Protein Stability Assay reaches completion, and no further change occurs. A p38a MAPK Protein Stability Assay was run with multiple titration curves of denaturant as a time course of room temperature incubation time. An equilibrium between soluble and precipitated protein occurs that does not continue to change with increasing time. This result is the same for different denaturants, but the time taken to reach completion varies between denaturants.
Figure 5: Protein Stability Assay demonstrating unfolding of protein in multiple denaturant types: The graph shows how the Protein Stability Assay works to generate protein unfolding curves in a range of denaturant types in varying proportions. The successful utilisation of different denaturants enables the most appropriate to be used to generate unfolding curves for the target of choice. A reduction in the concentration of chemical needed for the denaturation of more stable proteins can therefore prevent potential interference with the detection endpoint reagents.

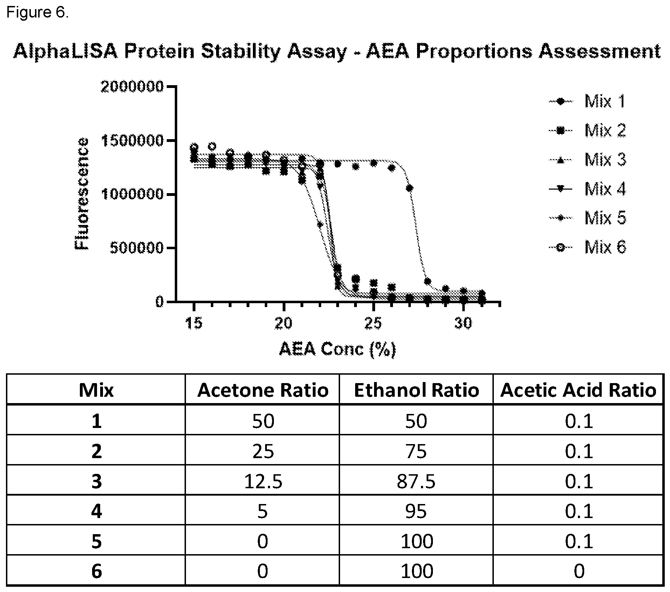

Figure 6: Effect of varying relative proportions of Acetone and Ethanol in AEA denaturant mix: Graph to show how varying the proportion of denaturant constituents (Ketone and Alcohol) within the mix, results in a shift in the concentration of denaturant required to unfold target protein in the Protein Stability Assay. Increased alcohol content relative to ketone results in a reduction of the concentration of denaturant required to unfold protein.

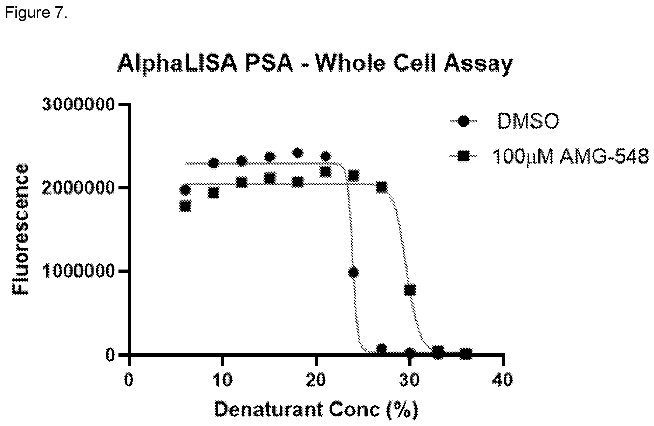

Figure 7: Whole Cell Protein Stability Assay: Graph to show how the Protein Stability Assay can be applied for use with whole cells as a source of target protein as opposed to isolated cell lysate cleared of membrane debris. Cells at a density of 1×10⁷/ml, with a final in well number of 50,000 were used in place of cell lysate. Stabilisation of p38a MAPK protein by AMG-548 compound can still be detected using the AlphaLISA^{®} endpoint.

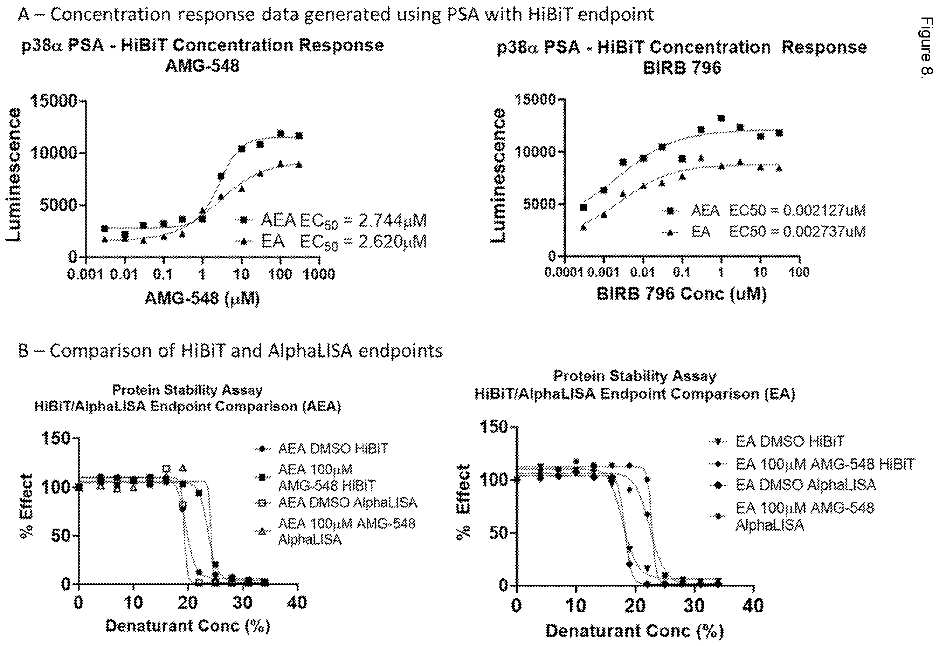

Figure 8: Protein Stability Assay using HiBiT^{®} technology endpoint: Demonstration of the use of lysate of HeLa p38α MAPK-linked HiBiT^{®} endogenously tagged cells in the protein stability assay. A: Concentration response curves generated using HiBiT^{®} endpoint data, showing clear dose dependent effect of AMG-548 and BIRB 796 compounds on protein unfolding. B: Graphs showing the comparison of compound stabilisation data between AlphaLISA^{®} and HiBiT^{®} endpoints in two different denaturants (AEA and EA). Graphs show high comparability between the two endpoint readouts when used in the same protein stability assay conditions.
Figure 9: Elucidation of compound MoA: Concentration response graphs to show how information about a compound's mode of action can be deduced by performing a protein stability assay at a range of denaturant concentrations with a dose response of compound concentrations (AMG-548 and BIRB 796). Testing a compound with a very slow off rate or an irreversible mode of action (in this case BIRB 796) results in very little difference in EC₅₀ values when run at different denaturant concentrations (B). When testing a reversible inhibitor (in this case AMG-548) there is a significant shift in EC₅₀ value at varying denaturant concentrations (A).

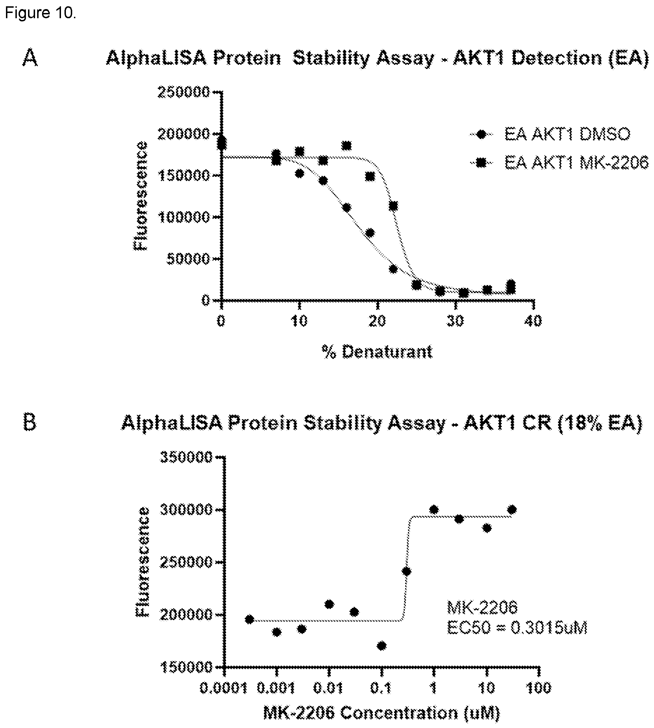

Figure 10: Use of Protein Stability Assay with an alternative target protein: A: Graph to show use of protein stability assay for detection of AKT1 in the presence and absence of compound (MK-2206) using the AlphaLISA^{®} endpoint. B: Concentration response plot showing effect of compound on AKT1 protein stability in a dose dependent manner at a fixed concentration of denaturant (18% EA) using the AlphaLISA^{®} endpoint.

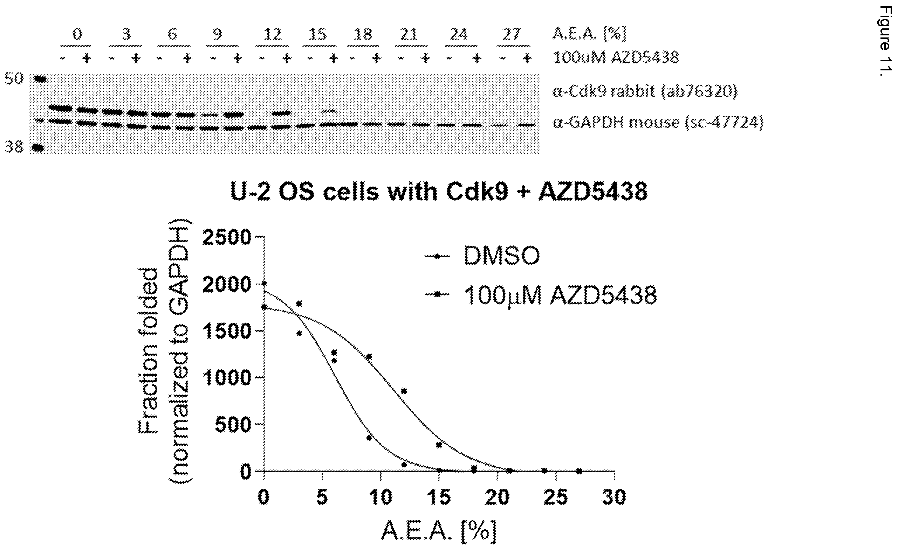

Figure 11: Protein Stability Assay using Western Blot endpoint: Protein samples in the presence or absence of 100 µM AZD5438 were treated with a gradient of AEA in 1.5ml tubes. After denaturation had occurred, samples were spun to remove aggregated protein and the remaining supernatant assayed by western blot to determine the relative amounts of CDK9 in each sample. Upper section: Western blot analysis of treated protein aliquots. When analysed by western blot CDK9 protein shows dose dependent unfolding. The addition of the inhibitor AZD5438 leads to stabilisation of CDK9 protein and results in a higher % of AEA being needed to unfold the protein. Lower section: Graph of quantified western blot bands (as shown in upper section). Graph shows dose dependent unfolding of protein with clear stabilisation by the addition of AZD5438.

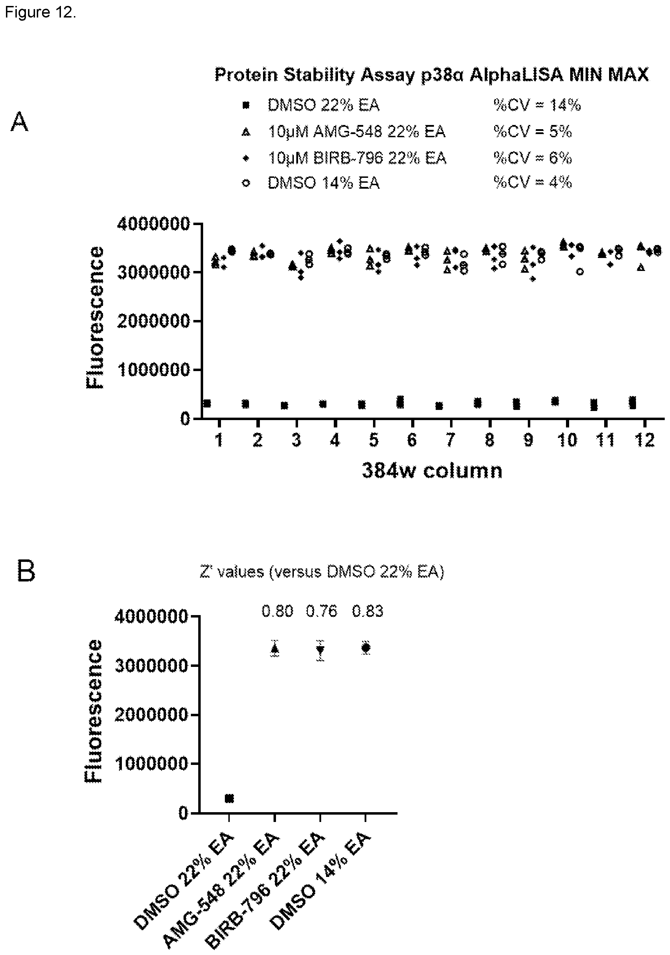

Figure 12: Demonstration of Chemical Protein Stability Assay single-well format suitable for high-throughput screening of chemical libraries to identify active compounds. (A) Individual results from p38a AlphaLISA^{®} assay 384w plate single wells showing Minimum basal signal (22% EA DMSO denatured target), Maximum Compound effect signal (22% EA with AMG-548 or BIRB-796-induced stabilisation of target). Maximum Compound effect produces signal comparable to undenatured target (14% EA), indicating optimal separation of Minimum and Maximum effect signals. (B) Mean and S.D. plot, together with calculated Z' statistics indicating a robust ability (Z' > 0.4) to differentiate active from inactive compounds in a screening format.

### EXAMPLES

### Example 1: p38α MAPK Target Protein Binding Assay Study Using HEK293T Cell Lysate.

The protein p38α MAPK was studied in the protein stability assay with an AlphaLISA^{®} endpoint (e.g. see workflow in Figure 1) using HEK293T cell lysate. Target protein in cell lysate was treated with or without the inhibitor AMG-548 at two concentrations followed by denaturation using a titration of AEA. The relative folded protein amounts under each treatment were measured by AlphaLISA^{®} to generate protein unfolding curves. Curves from compound treated samples were compared to those treated with DMSO only (Figure 2).

### Materials and Methods

Lysate from HEK293T cells grown in continuous culture at 5% CO₂/37°C in full growth media (RPMI (Gibco 11835-063), 10% FCS (Gibco F7524) 1% Glutamax (Gibco 35050-061)) was produced by growing cells in flasks to 85% confluence, removing from the surface using 3ml Tryple Select (Gibco 12563011), centrifuging (1000rpm for 4 minutes) to pellet, washing cell pellet two times in DPBS (Gibco 14190-094), resuspending in 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required.

To run the protein stability assay, an aliquot of HEK293T cell lysate at 4 µg/µl was thawed at room temperature and 2 µl added to each well of a 384-well White ProxiPlate (Perkin Elmer 6008280) containing 10 µM, 100 µM AMG-548 (Bio-Techne 3920/10) or an equivalent volume of DMSO dosed using the Labcyte ECHO. The contents of the plate were settled by a short pulse spin, sealed with a TopSeal-A Plus plate seal (Perkin Elmer 6050185), and the compound/lysate mixes incubated at ambient temperature for 30 minutes to 1 hour to allow potential target engagement by compound to occur. While incubating, fresh 100% AEA (Acetone (11443733), Ethanol (10437341) and Acetic Acid (10365020), all from Fisher Scientific in a ratio of 50:50:0.1) denaturant was made in a glass bottle ready for use. An aliquot of 100% AEA was diluted to 50% in DPBS (Gibco 14190-094) ready to produce titration curves of denaturant. An example titration series is shown below -

### Generic Denaturant Titration

| | **1** | **2** | **3** | **4** | **5** | **6** | **7** | **8** | **9** | **10** | **11** | **12** |
|---|---|---|---|---|---|---|---|---|---|---|---|---|
| Denaturant [%] | 0 | 4 | 7 | 10 | 13 | 16 | 19 | 22 | 25 | 28 | 31 | 34 |
| *Stock Conc Denaturant (%)* | *0* | *5* | *8.75* | *12.5* | *16.25* | *20* | *23.75* | *27.5* | *31.25* | *35* | *38.75* | *42.5* |
| 1x PBS Volume (µl) | 40 | 36 | 33 | 30 | 27 | 24 | 21 | 18 | 15 | 12 | 9 | 6 |
| 50% Denaturant Volume (µl) | 0 | 4 | 7 | 10 | 13 | 16 | 19 | 22 | 25 | 28 | 31 | 34 |
| Total Volume (µl) | 40 | 40 | 40 | 40 | 40 | 40 | 40 | 40 | 40 | 40 | 40 | 40 |

The dilution series to be added was made up as a 5/4 stock so that when 8 µl of denaturant was added to 2 µl lysate the final volume was 10 µl. The denaturant concentrations are as listed above. After compound/lysate incubation was complete 8 µl of the prepared AEA stock(s) was added to each well, the contents of the plate were settled by a short pulse spin, sealed, and then incubated with shaking at 800rpm for 120 minutes on a heated plate shaker set to 21°C. For signal detection an antibody detection mix was made up no more than 20 minutes prior to use. A solution containing 1x Immunoassay Buffer (Perkin Elmer AL000F), 0.05% SDS (Sigma 71736-100ml), 10 mg/ml rabbit acceptor beads (Perkin Elmer AL104C), 10 mg/ml mouse donor beads (Perkin Elmer AS104D), 0.2 nM (30 ng/ml) rabbit anti-p38α antibody (Abcam ab 170099) and 0.8 nM (120 ng/ml) mouse anti-p38α antibody (Abcam ab31828) was created in a suitable volume. 10 µl of antibody bead mix was added to each test well, the contents of the plate were settled by a short pulse spin, sealed, shaken gently for 1-2 minutes, wrapped in foil, and then incubated for 16-18 hours in the dark. After incubation the plate was read on a fluorescence detecting plate reader (Excitation 680 nm, Emission 615 nm) to determine well signals.

### Results

Treatment of cell lysate with AMG-548 at either 10 or 100 µM resulted in the stabilisation of p38α MAPK protein in a dose dependent manner (Figure 2). This is shown by a shift in protein denaturation curves when comparing compound treated samples with untreated (DMSO only) samples. This demonstrates that protein stabilisation, because of engagement by AMG-548, is detectable in lysate samples when using the protein stability assay.

### Example 2: p38α MAPK Target Protein Binding Assay Screening Study Using HEK293T Cell Lysate.

A set of inhibitor compounds (AMG-548 (Bio-Techne 3920/10), BIRB 796 (Apex Bio A5639-APE-10mg), SB203580 (Cambridge Bioscience S0500-5mg), SCIO 469 (Cambridge Bioscience CAY29484-5), VX 745 (Apollo Scientific BISN0180), ML 3403 (Bio-Techne 4586/10), EO 1428 (Bio-Techne 2908/10), AL 8697 (Bio-Techne 4753/10), U0126 (Cambridge Bioscience SM106-5), PD98059 (Stratech Scientific Limited A1663-APE-10mg), Wortmannin (Stratech Scientific Limited A8544-APE-5mg), Y27632 (Enzo Life Sciences LKT-Y1000-M005), HA1077 (Cambridge Bioscience SM49-10), Lapatinib (Stratech Scientific Limited A8218-APE-50mg), Gefitinib (Stratech Scientific Limited A8219-APE-100mg), Dasatinib (Apollo Scientific OR302638), Sunitinib (Stratech Scientific Limited B1045-APE-100mg) and Sorafenib (Cambridge Bioscience SM95-10)) with varying potencies against p38α MAPK protein were screened using the protein stability assay with an AlphaLISA^{®} endpoint (e.g. see workflow in Figure 1) to determine their ability to stabilise the target in HEK293T cell lysate. Target protein in cell lysate was treated with a set of inhibitors at a range of concentrations followed by denaturation using a fixed concentration of AEA or EA. The relative folded protein amounts under each treatment were measured by AlphaLISA^{®} to generate compound concentration response curves.

### Materials and Methods

Lysate from HEK293T cells grown in continuous culture at 5% CO₂/37°C in full growth media (RPMI (Gibco 11835-063), 10% FCS (Gibco F7524) 1% Glutamax (Gibco 35050-061)) was produced by growing cells in T175 flasks to 85% confluence, removing from the surface using 3ml Tryple Select (Gibco 12563011), centrifuging (1000rpm for 4 minutes) to pellet, washing cell pellet two times in DPBS (Gibco 14190-094), resuspending in 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required.

To run the protein stability assay, an aliquot of HEK293T cell lysate at 4 µg/µl was thawed at room temperature and 2 µl added to each well of duplicate 384-well White ProxiPlates (Perkin Elmer 6008280). The 384-well plates had been dosed with 10-point concentration response curves (one for each compound), in a range from 75 µM to 7.5 nM (with a different compound concentration in each well), of the p38α MAPK inhibiting compound set, using the Labcyte ECHO. The contents of the plate were settled by a short pulse spin, sealed with a TopSeal-A Plus plate seals (Perkin Elmer 6050185), and the compound/lysate mixes incubated at ambient temperature for 30 minutes to 1 hour to allow potential target engagement by compound to occur. While incubating, fresh 100% AEA (Acetone (11443733), Ethanol (10437341) and Acetic Acid (10365020), all from Fisher Scientific in a ratio of 50:50:0.1) or 100% EA (Ethanol (10437341) and Acetic Acid (10365020), all from Fisher Scientific in a ratio of 100:0.1) denaturants were made up in glass bottles ready for use. Aliquots of 100% AEA and 100% EA were diluted to 50% in DPBS (Gibco 14190-094) ready to produce a fixed concentration of denaturant. AEA was diluted in DPBS (Gibco 14190-094) to provide a final concentration of 22% and EA was diluted in DPBS (Gibco 14190-094) to give a final concentration of 20%.

The fixed denaturant concentration to be added was made up as a 5/4 stock so that when 8 µl of denaturant was added to 2 µl lysate the final volume was 10 µl and the denaturant concentrations were as listed above. After compound/lysate incubation was complete 8 µl of the prepared AEA or EA stock was added to each well of separate plates, the contents of the plates were settled by a short pulse spin, sealed, and then incubated with shaking at 800rpm for 120 minutes on a heated plate shaker set to 21°C. For signal detection an antibody detection mix was made up no more than 20 minutes prior to use. A solution containing 1x Immunoassay Buffer (Perkin Elmer AL000F), 0.05% SDS (Sigma 71736-100ml), 10 mg/ml rabbit acceptor beads (Perkin Elmer AL104C), 10 mg/ml mouse donor beads (Perkin Elmer AS104D), 0.2 nM (30 ng/ml) rabbit anti-p38α antibody (Abcam ab 170099) and 0.8 nM (120 ng/ml) mouse anti-p38α antibody (Abcam ab31828) was created in a suitable volume. 10 µl of antibody bead mix was added to each test well, the contents of the plates were settled by a short pulse spin, sealed, shaken gently for 1-2 minutes, wrapped in foil, and then incubated for 16-18 hours in the dark. After incubation the plates were read on a fluorescence detecting plate reader (Excitation 680 nm, Emission 615 nm) to determine well signals. Concentration response curves were produced from normalised data using the Genedata Screener data analysis package, and EC₅₀ values generated to allow data comparison between the two denaturant types.

### Results

Treatment of cell lysate with the set of inhibitors, and subsequent target stabilisation against protein denaturation, resulted in the generation of concentration response curves with a range of potencies against the p38α MAPK protein. Comparison of the data generated using AEA or EA showed that similar concentration response curve profiles are achieved when using either denaturant (Figure 3a). Repetition of the screening assay generates highly comparable replicate data (Figure 3b), demonstrating the robustness of the protein stability assay with regards to run-to-run variation. Comparison of the concentration response EC₅₀ values shows that highly comparable data can be achieved when using different denaturants (Figure 3c). This demonstrates the robustness of the protein stability assay in utilising a range of chemical denaturants.

### Example 3: p38α MAPK Target Protein Binding Assay Study Using HEK293T Cell Lysate to Assess Different Denaturant Mixtures, Different Relative Proportions of Mixture Constituents and the Length of Denaturation Incubation Time.

The protein p38α MAPK was studied in the protein stability assay with an AlphaLISA^{®} endpoint (e.g. see workflow in Figure 1) using HEK293T cell lysate. Target protein in cell lysate was denatured using titrations of a variety of mixtures and relative proportions. Time courses of denaturation incubation were also generated to monitor the effect of time on the protein stability assay. The relative folded protein amounts under each treatment were measured by AlphaLISA^{®} to generate protein unfolding curves to allow comparison of the different denaturant mixes.

### Materials and Methods

Lysate from HEK293T cells grown in continuous culture at 5% CO₂/37°C in full growth media (RPMI (Gibco 11835-063), 10% FCS (Gibco F7524) 1% Glutamax (Gibco 35050-061)) was produced by growing cells inT175 flasks to 85% confluence, removing from the surface using 3ml Tryple Select (Gibco 12563011), centrifuging (1000rpm for 4 minutes) to pellet, washing cell pellet two times in DPBS (Gibco 14190-094), resuspending 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required.

To run the protein stability assay, an aliquot of HEK293T cell lysate at 4 µg/µl was thawed at room temperature and 2 µl added to each test well of 384-well White ProxiPlates (Perkin Elmer 6008280) for each condition being assessed. The contents of the plate were settled by a short pulse spin. Fresh 100% AEA (Acetone (11443733), Ethanol (10437341) and Acetic Acid (10365020), all from Fisher Scientific in a ratio of 50:50:0.1), 100% BEA (Butanone (Honeywell 360473), Ethanol and Acetic Acid in a ratio of 50:50:0.1), 100% APA (Acetone, Propanol (Fisher 10254060) and Acetic Acid in a ratio of 50:50:0.1), 100% BPA (Butanone, Propanol and Acetic Acid in a ratio of 50:50:0.1), 100% EA (Ethanol and Acetic Acid in a ratio of 100:0.1), 100% PA (Propanol and Acetic Acid in a ratio of 100:0.1), 100% E (Ethanol) and 100% P (Propanol) denaturants were made in glass bottles ready for use. Mixtures of AEA in different proportions were also set up to assess the effect of altering the relative amounts of each component. The proportions used can be seen in Figure 6. An aliquot of each 100% mixture was diluted to 50% in DPBS (Gibco 14190-094) ready to produce titration curves of each denaturant. An example titration series is shown below -

### Generic Denaturant Titration

| | **1** | **2** | **3** | **4** | **5** | **6** | **7** | **8** | **9** | **10** | **11** | **12** |
|---|---|---|---|---|---|---|---|---|---|---|---|---|
| Denaturant [%] | 0 | 4 | 7 | 10 | 13 | 16 | 19 | 22 | 25 | 28 | 31 | 34 |
| *Stock Conc Denaturant (%)* | *0* | *5* | *8.75* | *12.5* | *16.25* | *20* | *23.75* | *27.5* | *31.25* | *35* | *38.75* | *42.5* |
| 1x PBS Volume (µl) | 40 | 36 | 33 | 30 | 27 | 24 | 21 | 18 | 15 | 12 | 9 | 6 |
| 50% Denaturant Volume (µl) | 0 | 4 | 7 | 10 | 13 | 16 | 19 | 22 | 25 | 28 | 31 | 34 |
| Total Volume (µl) | 40 | 40 | 40 | 40 | 40 | 40 | 40 | 40 | 40 | 40 | 40 | 40 |

The dilution series to be added was made up as a 5/4 stock so that when 8 µl of denaturant was added to 2 µl lysate the final volume was 10 µl. The denaturant concentrations are as listed above. After compound/lysate incubation was complete 8 µl of each of the prepared denaturant stocks was added to each well as required, the contents of the plate were settled by a short pulse spin, sealed, and then incubated with shaking at 800rpm for 120 minutes on a heated plate shaker set to 21°C. For the denaturant incubation time course plate, multiple titrations of AEA and EA were created and incubated for different times from 20 to 120 minutes). For the denaturant mixture comparison plate titration curves were set up for each mixture. For the denaturant proportions plate titration curves of AEA in varying proportions were produced. For signal detection an antibody detection mix was made up no more than 20 minutes prior to use. A solution containing 1x Immunoassay Buffer (Perkin Elmer AL000F), 0.05% SDS (Sigma 71736-100ml), 10 mg/ml rabbit acceptor beads (Perkin Elmer AL104C), 10 mg/ml mouse donor beads (Perkin Elmer AS104D), 0.2 nM (30 ng/ml) rabbit anti-p38α antibody (Abcam ab 170099) and 0.8 nM (120 ng/ml) mouse anti-p38α antibody (Abcam ab31828) was created in a suitable volume. 10 µl of antibody bead mix was added to each test well, the contents of the plate were settled by a short pulse spin, sealed, shaken gently for 1-2 minutes, wrapped in foil, and then incubated for 16-18 hours in the dark. After incubation the plates were read on a fluorescence detecting plate reader (Excitation 680 nm, Emission 615 nm) to determine well signals.

### Results

Varying the length of denaturation time shows that if allowed to run, a point can be reached at which the Protein Stability Assay reaches completion, and no further change occurs (Figure 4). An equilibrium between soluble and precipitated protein occurs that does not continue to change with increasing time. This result was the same for different denaturants, but the time taken to reach completion varied between denaturants. Figure 5 illustrates that it is possible to use different denaturant types to generate protein unfolding curves for the same target. The successful utilisation of different denaturants enables the most appropriate mixture to be used to generate unfolding curves for the target of choice. A reduction in the concentration of chemical needed for the denaturation of more stable proteins can therefore prevent potential interference with the detection endpoint reagents. Figure 6 shows the effect of varying relative proportions of Acetone and Ethanol in the AEA denaturant mixture. Varying the proportion of denaturant constituents (Ketone and Alcohol) within the mixture results in a shift in the concentration of denaturant required to unfold target protein in the Protein Stability Assay. An increase in the proportion of alcohol relative to ketone in the denaturant mixture results in an increase in the potency of the mixture. The increased potency leads to more effective protein unfolding at a lower concentration of denaturant mixture. This highlights that the alcohol component of the denaturant mixture has the greatest influence on protein denaturation.

### Example 4: p38α MAPK Target Protein Binding Assay Study Using Intact HEK293T Cells as a starting material.

The protein p38α MAPK was studied in the protein stability assay with an AlphaLISA^{®}endpoint (e.g. see workflow in Figure 1) using intact HEK293T cells as a starting material. Cells were treated with or without the inhibitor AMG-548 followed by denaturation using a titration of EA. The relative folded protein amounts under each treatment were measured by AlphaLISA^{®} to generate protein unfolding curves. Curves from compound treated samples were compared to those treated with DMSO only.

HEK293T cells were grown in media (RPMI (Gibco 11835-063), 10% FCS (Gibco F7524) 1% Glutamax (Gibco 35050-061)) to approximately 85% confluence in a flask before harvesting from the surface using 3ml Tryple Select (Gibco 12563011), washing cell pellet once in DPBS (Gibco 14190-094) and resuspending in media. Cells were counted and diluted in media to a density of 1x10⁷ cell/ml.

To run the protein stability assay 5 µl of cells at a density of 1x10⁷ cell/ml was added to each well of a 384-well White ProxiPlate (Perkin Elmer 6008280) containing 100 µM AMG-548 (Bio-Techne 3920/10) or an equivalent volume of DMSO dosed using the Labcyte ECHO. The contents of the plate were settled by a short pulse spin, sealed with a TopSeal-A Plus plate seal (Perkin Elmer 6050185) and the compound/lysate mixes incubated at ambient temperature for 30 minutes to 1 hour to allow potential target engagement by compound to occur. While incubating, fresh 100% EA (Ethanol (10437341) and Acetic Acid (10365020), all from Fisher Scientific in a ratio of 100:0.1) denaturant was made in a glass bottle ready for use. 100% EA was used to produce a titration curve of denaturant. An example titration series is shown below -

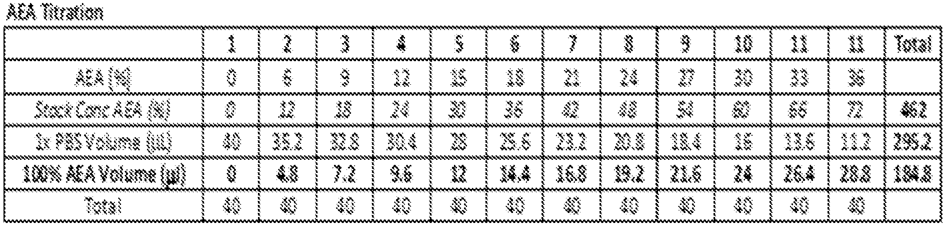

The dilution series to be added was made up as a 2x stock so that when 5 µl of denaturant was added to 5 µl lysate the final volume was 10µl. The denaturant concentrations were as listed above. After compound/lysate incubation was complete 5 µl of the prepared EA stock was added to each well, the contents of the plate were settled by a short pulse spin, sealed and then incubated with shaking at 800rpm for 120 minutes on a heated plate shaker set to 21°C. For signal detection an antibody detection mix is made up no more than 20 minutes prior to use. A solution containing 1x Immunoassay Buffer (Perkin Elmer AL000F), 0.05% SDS (Sigma 71736-100ml), 10 mg/ml rabbit acceptor beads (Perkin Elmer AL104C), 10 mg/ml mouse donor beads (Perkin Elmer AS104D), 0.2 nM (30 ng/ml) rabbit anti-p38α antibody (Abcam ab 170099) and 0.8 nM (120 ng/ml) mouse anti-p38α antibody (Abcam ab31828) was created in a suitable volume. 10 µl of antibody bead mix was added to each test well, the contents of the plate were settled by a short pulse spin, sealed, shaken gently for 1-2 minutes, wrapped in foil, and then incubated for 16-18 hours in the dark. After incubation the plate was read on a fluorescence detecting plate reader (Excitation 680 nm, Emission 615 nm) to determine well signals.

### Results

Treatment of intact HEK293T cells with 10 µM AMG-548 resulted in the stabilisation of p38α MAPK protein compared to DMSO only treatment (Figure 7). A shift in the protein denaturation curves when comparing compound treated samples with untreated (DMSO only) samples. This demonstrates that protein stabilisation because of engagement of AMG-548 is detectable in a protein stability assay using intact cells as a starting material. The denaturation curve profiles and shift due to stabilisation are comparable to those generated using cell lysate.

### Example 5: Detection of increased protein stability for a target protein using the Protein Stability Assay with a Western blot endpoint.

The protein CDK9 was studied in the protein stability assay using U-2 OS cell lysate. Cell lysate was treated with or without the inhibitor AZD5438 followed by denaturation using a titration of AEA and the relative folded protein amounts under each treatment measured by Western blot to generate protein unfolding curves. A curve from compound treated samples was compared to those treated with DMSO only.

### Materials and Methods

Lysate from U-2 OS cells grown in continuous culture at 5% CO₂/37°C in full growth media (RPMI (Gibco 11835-063), 10% FCS (Gibco F7524) 1% Glutamax (Gibco 35050-061)) was produced by growing cells in T175 flasks to 85% confluence, removing from the surface using 3ml Tryple Select (Gibco 12563011), centrifuging (1000rpm for 4 minutes) to pellet, washing cell pellet two times in DPBS (Gibco 14190-094), resuspending 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required.

To run the protein stability assay, an aliquot of HEK293T cell lysate at 4 µg/µl was thawed at room temperature and 220 µl treated with 2.2 µl of AZD5438 (Sigma SML1855-5mg) at a concentration of 10 µM or DMSO in 1.5 ml tubes. Lysate/compound mixes were incubated for 30-60 minutes at room temperature. While incubating, fresh 100% AEA (Acetone (11443733) Ethanol (10437341) and Acetic Acid (10365020), all from Fisher Scientific in a ratio of 50:50:0.1) denaturant was made in a glass bottle ready for use. An aliquot of 100% AEA was diluted to 50% in DPBS (Gibco 14190-094) ready to produce either titration curves or a fixed concentration of denaturant. An example titration series is shown below and was made up in 1.5ml tubes with a final volume of 50 µl -

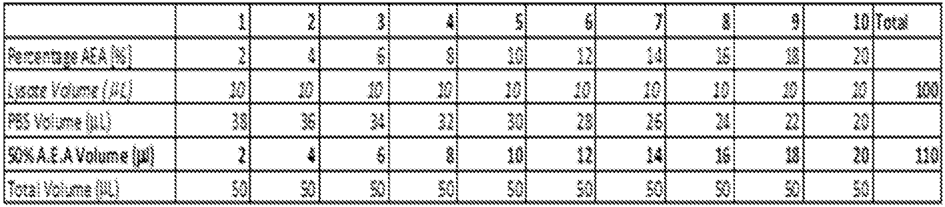

Once created 10 µl of lysate was added to each tube and then the tubes were incubated with shaking at 800rpm for 120 minutes at 21°C. After incubation, tubes were centrifuged for 15-20 minutes at 20,000g/4°C to pellet precipitated/aggregated protein. 27 µl of each supernatant was added to 9 µl loading dye (4x NuPAGE LDS Sample buffer containing 40mM DTT added fresh - NP0007) in 1.5 ml tubes. All tubes were incubated at 70°C for 10 minutes and then spun briefly to settle the contents. 12 µl of sample from each tube was loaded onto a NuPAGE 4-12% Bis-Tris, 26-well gel (Invitrogen WG1403BX10). The Western blot gel was run in 1x MOPS buffer (from 20x stock) at 200V for 50 minutes. Gel contents were transferred to a Western blot nitrocellulose membrane using the Invitrogen iBlot 2 Gel Transfer Device. The nitrocellulose membrane was cut into appropriate regions for detection of proteins and sections were blocked in TBS Intercept blocking buffer (Li-Cor 927-60001) without Tween-20 for 1-2 hours at room temperature with rocking. Primary antibodies (Anti-CDK9 rabbit (Abcam ab76320 1:5000) target protein antibody and an anti-GAPDH mouse (Santa Cruz sc-47724 1:500) housekeeping protein antibody for normalisation of protein amount) were added to appropriate membrane sections in a solution of Intercept containing 0.2% Tween-20 and incubated on a rocker over-night at 4°C. After incubation blots were washed every 5 minutes for 30 minutes in TBS containing 0.05% Tween with rocking. After washing, blots were incubated with secondary antibodies (IRDye^{®} 800CW Goat anti-Rabbit IgG Secondary Antibody (Li-Cor 926-32211 1:5000) or IRDye^{®} 680RD Goat anti-Mouse IgG Secondary Antibody (Li-Cor 926-68070 1:5000) for 1 hour at room temperature with rocking. After incubation blots were washed every 5 minutes for 25 minutes in TBS containing 0.05% Tween and then one final wash in TBS alone, all with rocking. Bands on blots were detected and quantified using the Li-Cor Odyssey CLx imaging system.

### Results

Treatment of cell lysate with 100 µM AZD5438 resulted in increased stabilisation of CDK9 protein when subjected to chemical denaturation by AEA (Figure 11). This effect is clear when comparing AEA titration curves for compound treated and untreated samples as a shift occurs showing that a greater percentage of AEA is required to unfold the compound treated protein.

### Example 6: p38α MAPK Target Protein Binding Assay Concentration Response Study Using HEK293T Cell Lysate to Elucidate Compound Mode of Action.

The known p38α MAPK inhibitors AMG-548 (Bio-Techne 3920/10) and BIRB 796 (Apex Bio A5639-APE-10mg) were screened at various denaturant concentrations using the protein stability assay with an AlphaLISA^{®} endpoint (e.g. see workflow in Figure 1). This was carried out to determine how the curve shapes and EC50 values of the compounds changed depending on whether they have reversible (AMG-548) or irreversible (BIRB 796) binding modes of action against p38α MAPK. Target protein in cell lysate was treated with the inhibitors at a dose response of concentrations followed by denaturation using a various fixed concentrations of EA. The relative folded protein amounts under each treatment were measured by AlphaLISA^{®} to generate compound concentration response curves.

### Methods and Materials

Lysate from HEK293T cells grown in continuous culture at 5% CO₂/37°C in full growth media (RPMI (Gibco 11835-063), 10% FCS (Gibco F7524) 1% Glutamax (Gibco 35050-061)) was produced by growing cells in T175 flasks to 85% confluence, removing from the surface using 3 ml Tryple Select (Gibco 12563011), centrifuging (1000rpm for 4 minutes) to pellet, washing cell pellet two times in DPBS (Gibco 14190-094), resuspending 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required.

To run the protein stability assay, an aliquot of HEK293T cell lysate at 4 µg/µl was thawed at room temperature and 2 µl added to each well of duplicate 384-well White ProxiPlates (Perkin Elmer 6008280) containing concentration response curves of AMG-548 (Bio-Techne 3920/10) and BIRB 796 (Apex Bio A5639-APE-10mg) dosed using the Labcyte ECHO. The contents of the plate were settled by a short pulse spin, sealed with a TopSeal-A Plus plate seals (Perkin Elmer 6050185), and the compound/lysate mixes incubated at ambient temperature for 30 minutes to 1 hour to allow potential target engagement by compound to occur. While incubating, fresh 100% EA (Ethanol (10437341) and Acetic Acid (10365020), all from Fisher Scientific in a ratio of 100:0.1) denaturant was made up in a glass bottle ready for use. An aliquot of 100% EA was diluted to 50% in DPBS (Gibco 14190-094) ready to produce a various fixed concentrations of denaturant. EA was diluted in DPBS (Gibco 14190-094) to provide a final concentration of 18%, 19%, 20% or 21%.

The fixed denaturant concentration to be added was made up as a 5/4 stock so that when 8 µl of denaturant was added to 2 µl lysate the final volume was 10 µl and the denaturant concentrations were as listed above. After compound/lysate incubation was complete 8 µl of the prepared EA stock was added to each appropriate well of the plate, the contents of the plate were settled by a short pulse spin, sealed, and then incubated with shaking at 800rpm for 120 minutes on a heated plate shaker set to 21°C. For signal detection an antibody detection mix was made up no more than 20 minutes prior to use. A solution containing 1x Immunoassay Buffer (Perkin Elmer AL000F), 0.05% SDS (Sigma 71736-100ml), 10 mg/ml rabbit acceptor beads (Perkin Elmer AL104C), 10 mg/ml mouse donor beads (Perkin Elmer AS104D), 0.2 nM (30 ng/ml) rabbit anti-p38α antibody (Abcam ab 170099) and 0.8 nM (120 ng/ml) mouse anti-p38α antibody (Abcam ab31828) was created in a suitable volume. 10 µl of antibody bead mix was added to each test well, the contents of the plates were settled by a short pulse spin, sealed, shaken gently for 1-2 minutes, wrapped in foil, and then incubated for 16-18 hours in the dark. After incubation the plates were read on a fluorescence detecting plate reader (Excitation 680 nm, Emission 615 nm) to determine well signals.

### Results

The compound concentration response curves shown in Figure 9A for the reversible binding inhibitor AMG-548 highlight that EC₅₀ values and curve shape change significantly when compound concentration response curves are compared across multiple denaturant concentrations. The compound concentration response curves shown in Figure 9B for the irreversible binding inhibitor BIRB 796 highlight that the EC₅₀ values and curve shapes remain more consistent when compound concentration response curves are compared across multiple denaturant concentrations. This shows that by use of an adapted protein stability assay to compare compound concentration response curves generated across a range of denaturant concentrations it is possible to distinguish between reversible and irreversible modes of action of the compound. This method can be applied to other target proteins by choosing a range of denaturant concentrations that span the denaturant concentrations where a compound-induced stabilisation shift is evident in the protein unfolding curve, as defined by denaturant titration experiments (e.g. if a stabilisation curve shift of target protein occurs between 19 and 20% EA, compound concentration response curves would be generated using denaturant concentrations of 18%, 19%, 20% and 21% EA).

### Example 7: p38α MAPK Target Protein Binding Assay Study Using HeLa p38α HiBiT^{®} Cell Lysate and a HiBiT^{®} Endpoint in comparison with an AlphaLISA^{®} Endpoint.

The protein p38α MAPK was studied in the protein stability assay with a HiBiT^{®} endpoint or an AlphaLISA^{®} endpoint using HeLa p38α HiBiT^{®} cell lysate. Target protein in cell lysate was treated with or without the inhibitors AMG-548 and BIRB 796 either at a single dose (AMG-548) or in a concentration response (AMG-548 and BIRB 796) of compound. Treatment was followed by denaturation using a titration of AEA or EA (when using a single dose of compound), or at a fixed dose of AEA or EA (when using a concentration response of compound). The relative folded protein amounts under each treatment were measured by HiBiT^{®} or AlphaLISA^{®} to generate protein unfolding curves. Curves from the different conditions and endpoints were compared to determine whether the use of different endpoint technologies could produce comparable data.

### Materials and Methods

Hela cells expressing the p38α target protein as a C-terminal fusion with a small peptide that includes the Promega HiBit^{®} amino acid sequence were used as the starting material for lysate generation. This cell line was generated based on the methods published by Promega (e.g. Schwinn et al., Sci Rep 10, 8953 (2020). https://doi.org/10.1038/s41598-020-65832-1). Lysate from HeLa p38α HiBiT^{®} cells grown in continuous culture at 5% CO₂/37°C in full growth media (RPMI (Gibco 11835-063), 10% FCS (Gibco F7524) 1% Glutamax (Gibco 35050-061)) was produced by growing cells in T175 flasks to 85% confluence, removing from the surface using 3 ml Tryple Select (Gibco 12563011), centrifuging (1000rpm for 4 minutes) to pellet, washing cell pellet two times in DPBS (Gibco 14190-094), resuspending 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required.

Standard lysate-based protein stability assays, as described previously, to generate denaturant titration curves (with and without 100 µM AMG-548) or compound concentration response curves of AMG548 (Bio-Techne 3920/10) and BIRB 798 (Apex Bio A5639-APE-10mg) at a fixed dose of denaturant were run using AEA and EA with a HiBiT^{®} detection endpoint or an AlphaLISA^{®} endpoint to allow comparison.

For HiBiT^{®} signal detection HiBiT^{®} substrate detection mix was made up no more than 20 minutes prior to use using the Promega Nano Glo HiBiT^{®} Lytic Detection Kit (N3030). A solution containing 1:100 LgBiT^{®} Protein and 1:100 Nano-Glo HiBiT^{®} Lytic Substrate in PBS was created in a suitable volume. 10 µl of substrate detection mix was added to each test well, the contents of the plate were settled by a short pulse spin, sealed, shaken gently for more than 15 minutes and then incubated for 80-120 minutes at room temperature. After incubation the plate was read on a luminescence detecting plate reader (3600 gain, 470-480 emission) to determine well signals.

For AlphaLISA^{®} signal detection an antibody detection mix is made up no more than 20 minutes prior to use. A solution containing 1x Immunoassay Buffer (Perkin Elmer AL000F), 0.05% SDS (Sigma 71736-100ml), 10 mg/ml rabbit acceptor beads (Perkin Elmer AL104C), 10 mg/ml mouse donor beads (Perkin Elmer AS104D), 0.2 nM (30 ng/ml) rabbit anti-p38α antibody (Abcam ab 170099) and 0.8 nM (120 ng/ml) mouse anti-p38α antibody (Abcam ab31828) was created in a suitable volume. 10 µl of antibody bead mix was added to each test well, the contents of the plate were settled by a short pulse spin, sealed, shaken gently for 1-2 minutes, wrapped in foil, and then incubated for 16-18 hours in the dark. After incubation the plate was read on a fluorescence detecting plate reader (Excitation 680 nm, Emission 615 nm) to determine well signals.

### Results

Figure 8A shows that compound concentration response curves for AMG-548 and BIRB 796 against the p38α MAPK target protein can be generated using the HiBiT^{®} assay and that they are comparable to those from AlphaLISA^{®} assays. This highlights that different endpoints can be used to create the equivalent data which increases the applications of this assay. Figure 8B clearly demonstrates that the use of HiBiT^{®} or AlphaLISA^{®} endpoints can generate near identical protein stabilisation data across a range of denaturants. This example uses 100 µM AMG-548 to stabilise the p38α MAPK target protein and shows a clear shift between the titration curves of compound treated samples compared with DMSO only treated samples.

### Example 8: AKT1 Target Protein Binding Assay Study Using HEK293T Cell Lysate.

The protein AKT1 was studied in the protein stability assay with an AlphaLISA^{®} endpoint (e.g. see workflow in Figure 1) using HEK293T cell lysate. Target protein in cell lysate was treated with or without the inhibitor MK-2206 at 10 µM followed by denaturation using a titration of EA. The relative folded protein amounts under each treatment were measured by AlphaLISA^{®} to generate protein unfolding curves. Curves from compound treated samples were compared to those treated with DMSO only (Figure 10A). Target protein in cell lysate was subsequently treated with a concentration response of MK-2206 followed by denaturation using a titration of EA. The relative folded protein amounts under each treatment were measured by AlphaLISA^{®} to generate concentration response curves (Figure 10B).

### Materials and Methods

Lysate from HEK293T cells grown in continuous culture at 5% CO₂/37°C in full growth media (RPMI (Gibco 11835-063), 10% FCS (Gibco F7524) 1% Glutamax (Gibco 35050-061)) was produced by growing cells in flasks to 85% confluence, removing from the surface using 3ml Tryple Select (Gibco 12563011), centrifuging (1000rpm for 4 minutes) to pellet, washing cell pellet two times in DPBS (Gibco 14190-094), resuspending in 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required.

To run the protein stability assay, an aliquot of HEK293T cell lysate at 4 µg/µl was thawed at room temperature and 2 µl added to each well of a 384-well White ProxiPlate (Perkin Elmer 6008280) containing 10 µM MK-2206 (LKT Laboratories M400220) or an equivalent volume of DMSO dosed using the Labcyte ECHO. The contents of the plate were settled by a short pulse spin, sealed with a TopSeal-A Plus plate seal (Perkin Elmer 6050185), and the compound/lysate mixes incubated at ambient temperature for 30 minutes to 1 hour to allow potential target engagement by compound to occur. While incubating, fresh 100% EA (Ethanol (10437341) and Acetic Acid (10365020), all from Fisher Scientific in a ratio of 100:0.1) denaturant was made in a glass bottle ready for use. An aliquot of 100% EA was diluted to 50% in DPBS (Gibco 14190-094) ready to produce titration curves of denaturant. An example titration series is shown below -

### Generic Denaturant Dilution

| | **1** | **2** | **3** | **4** | **5** | **6** | **7** | **8** | **9** | **10** | **11** | **12** |
|---|---|---|---|---|---|---|---|---|---|---|---|---|
| Deanturant [%] | 0 | 7 | 10 | 13 | 16 | 19 | 22 | 25 | 28 | 31 | 34 | 37 |
| *Stock Conc Denaturant (%)* | *0* | *8.75* | *12.5* | *16.25* | *20* | *23.75* | *27.5* | *31.25* | *35* | *38.75* | *42.5* | *46.25* |
| 1x PBS Volume (µl) | 40 | 33 | 30 | 27 | 24 | 21 | 18 | 15 | 12 | 9 | 6 | 3 |
| 50% Denaturant Volume (µl) | 0 | 7 | 10 | 13 | 16 | 19 | 22 | 25 | 28 | 31 | 34 | 37 |
| Total Volume (µl) | 40 | 40 | 40 | 40 | 40 | 40 | 40 | 40 | 40 | 40 | 40 | 40 |

The dilution series to be added was made up as a 5/4 stock so that when 8 µl of denaturant was added to 2 µl lysate the final volume was 10 µl. The denaturant concentrations are as listed above. After compound/lysate incubation was complete 8 µl of the prepared EA stock(s) was added to each well, the contents of the plate were settled by a short pulse spin, sealed, and then incubated with shaking at 800rpm for 120 minutes on a heated plate shaker set to 21°C. For AKT1 signal detection an antibody detection mix was made up no more than 20 minutes prior to use. A solution containing 1x Immunoassay Buffer (Perkin Elmer AL000F), 0.05% SDS (Sigma 71736-100ml), 10 µg/ml rabbit acceptor beads (Perkin Elmer AL104C), 10 µg/ml mouse donor beads (Perkin Elmer AS104D), 1:2000 rabbit anti-AKT (pan) antibody (Cell Signaling Technology CST 4691T) and 1:5000 mouse anti-AKT (5G3) antibody (Cell Signaling Technology CST 2966S) was created in a suitable volume. 10 µl of antibody bead mix was added to each test well. After detection reagent addition the contents of the plate were settled by a short pulse spin, sealed, shaken gently for 1-2 minutes, wrapped in foil, and then incubated for 16-18 hours in the dark. After incubation the plate was read on a fluorescence detecting plate reader (Excitation 680 nm, Emission 615 nm) to determine well signals.

From the denaturant curves generated a concentration of 18% EA was chosen to be used in an experiment to generate MK-2206 concentration response curves. To run the protein stability assay, an aliquot of HEK293T cell lysate at 4 µg/µl was thawed at room temperature and 2 µl added to each well of duplicate 384-well White ProxiPlates (Perkin Elmer 6008280) containing concentration response curves of MK-2206, dosed using the Labcyte ECHO. The contents of the plate were settled by a short pulse spin, sealed with a TopSeal-A Plus plate seals (Perkin Elmer 6050185), and the compound/lysate mixes incubated at ambient temperature for 30 minutes to 1 hour to allow potential target engagement by compound to occur. While incubating, fresh 100% EA (Ethanol (10437341) and Acetic Acid (10365020), all from Fisher Scientific in a ratio of 100:0.1) denaturant was made up in a glass bottle ready for use. An aliquot 100% EA was diluted to 50% in DPBS (Gibco 14190-094) ready to produce a fixed concentration of denaturant. EA was diluted in DPBS (Gibco 14190-094) to provide a final concentration of 18%.

The fixed denaturant concentration to be added was made up as a 5/4 stock so that when 8 µl of denaturant was added to 2 µl lysate the final volume was 10 µl and the denaturant concentrations were as listed above. After compound/lysate incubation was complete 8 µl of the prepared AEA or EA stock was added to each well of separate plates, the contents of the plates were settled by a short pulse spin, sealed, and then incubated with shaking at 800rpm for 120 minutes on a heated plate shaker set to 21°C. For AKT1 signal detection an antibody detection mix was made up no more than 20 minutes prior to use. A solution containing 1x Immunoassay Buffer (Perkin Elmer AL000F), 0.05% SDS (Sigma 71736-100ml), 10 µg/ml rabbit acceptor beads (Perkin Elmer AL104C), 10 µg/ml mouse donor beads (Perkin Elmer AS104D), 1:2000 rabbit anti-AKT (pan) antibody (Cell Signaling Technology CST 4691T) and 1:5000 mouse anti-AKT (5G3) antibody (Cell Signaling Technology CST 2966S) was created in a suitable volume. 10 µl of antibody bead mix was added to each test well. After detection reagent addition the contents of the plate were settled by a short pulse spin, sealed, shaken gently for 1-2 minutes, wrapped in foil, and then incubated for 16-18 hours in the dark. After incubation the plate was read on a fluorescence detecting plate reader (Excitation 680 nm, Emission 615 nm) to determine well signals.

### Results

Treatment of cell lysate with MK-2206 at 10 µM resulted in the stabilisation of AKT1 protein (Figure 10A). This is shown by a shift in protein denaturation curves when comparing compound treated samples with untreated (DMSO only) samples. This demonstrates that protein stabilisation, because of engagement by MK-2206, is detectable in lysate samples when using the protein stability assay. Analysis of the data resulted in the choice of 18% EA as a fixed concentration of denaturant to be used in a subsequent concentration response assay. AKT1 protein stabilisation by MK-2206 in a dose dependent manner was demonstrated through the generation of a concentration response curve (Figure 10B). This result demonstrates the application of the protein stability assay to multiple targets.

### Example 9: detection of active compound in a single-well per test compound high-throughput screen.

Use of the Chemical Protein Stability Assay in a single-well screening format to identify active compounds or hits, such as during high-throughput screening of chemical libraries for drug discovery was carried out. The results are shown in Figure 11. Suitably, when run in a single-well per test compound format, the signals generated by active versus inactive compounds in the assay are sufficiently separated so that these activities can be reliably distinguished, such as shown in Figure 11B.

### Materials and Methods

Lysate from HEK293T cells grown in continuous culture at 5% CO₂/37°C in full growth media (RPMI (Gibco 11835-063), 10% FCS (Gibco F7524) 1% Glutamax (Gibco 35050-061)) was produced by growing cells in flasks to 85% confluence, removing from the surface using 3ml Tryple Select (Gibco 12563011), centrifuging (1000rpm for 4 minutes) to pellet, washing cell pellet two times in DPBS (Gibco 14190-094), resuspending in 1 ml of DPBS (Gibco 14190-094) containing 1x HALT Protease and Phosphatase inhibitor cocktail (Thermo Scientific 78440) per flask and lysing by three cycles of a liquid nitrogen freeze followed by thawing at room temperature. Cell debris was removed by centrifugation at 20,000g/4°C for 15 minutes, supernatant diluted in DPBS (Gibco 14190-094) containing 1x HALT to a concentration of 4µg/µl after protein determination using a Bradford Reagent Assay (Quick Start^{™} Bradford Protein Assay Kit - Bio Rad 5000202) and aliquoted before freezing at -80°C until required. To run the protein stability assay, an aliquot of HEK293T cell lysate at 4 µg/µl was thawed at room temperature and 2 µl added to each well of a 384-well White ProxiPlate (Perkin Elmer 6008280) containing 10 µM AMG-548 (Bio-Techne 3920/10), BIRB-796 (Apex Bio A5639-APE-10mg) or an equivalent volume of DMSO dosed using the Labcyte ECHO. The contents of the plate were settled by a short pulse spin, sealed with a TopSeal-A Plus plate seal (Perkin Elmer 6050185), and the compound/lysate mixes incubated at ambient temperature for 30 minutes to 1 hour to allow potential target engagement by compound to occur.

While incubating, fresh 100% EA (Ethanol (Fisher Scientific 10437341) and Acetic Acid (Fisher Scientific 10365020) in a ratio of 100:0.1) denaturant was made in a glass bottle ready for use. An aliquot of 100% EA was diluted to 50% in DPBS and then further diluted to working stocks (17.5% and 27.5%) of 5/4 the desired final concentrations. After incubation, 8µl of denaturant was added to each well, to give a final volume of 10µl and final denaturant concentrations of 14% and 22%. The contents of the plate were settled by a short pulse spin, sealed, and then incubated with shaking at 800rpm for 120 minutes on a heated plate shaker set to 21°C.

For signal detection an antibody detection mix was made up no more than 20 minutes prior to use. A solution containing 1x Immunoassay Buffer (Perkin Elmer AL000F), 0.05% SDS (Sigma 71736-100ml), 10 mg/ml rabbit acceptor beads (Perkin Elmer AL104C), 10 mg/ml mouse donor beads (Perkin Elmer AS104D), 0.2 nM (30 ng/ml) rabbit anti-p38α antibody (Abcam ab 170099) and 0.8 nM (120 ng/ml) mouse anti-p38α antibody (Abcam ab31828) was created in a suitable volume. 10 µl of antibody bead mix was added to each test well, the contents of the plate were settled by a short pulse spin, sealed, shaken gently for 1-2 minutes, wrapped in foil, and then incubated for 16-18 hours in the dark. After incubation the plate was read on a fluorescence detecting plate reader (Excitation 680 nm, Emission 615 nm) to determine well signals.

### Results

Individual wells containing 22% EA and DMSO vehicle produce a low, consistent (14% Coefficient of Variation Minimum signal while individual wells containing active compounds (22% EA with AMG-548 or BIRB-796) produce a consistent (5-6% CV), higher Maximum effect signal that shows clear differentiation between minimum and maximum signals (Figure 11). Calculation of Z' statistics (Z' > 0.4) indicates that active compounds could be reliably distinguished from inactive compounds in a single-well per test compound screen.

## Claims

1. A method of identifying whether a protein target and a compound of interest bind to one another, the method comprising, in order, the steps of:
i) exposing the protein target to the compound of interest for a time sufficient to allow binding of the compound of interest to the protein target;
ii) contacting the protein target with a chemical denaturing agent to produce a denaturing mixture;
iii) assaying the products of the denaturing mixture to determine the proportion of the protein target in its folded state and/or the proportion of protein in its denatured state;
wherein a change in the proportion of protein in its folded state and/or the proportion of protein in its denatured state as compared to a suitable control indicates that the protein target and compound of interest bind to one another, and wherein step ii) of the method is conducted at a substantially constant temperature; wherein steps ii) and iii) are conducted in the same vessel; and wherein the chemical denaturing agent comprises at least one of: an acid; an alcohol; and a ketone.

2. The method of claim 1, wherein each of steps i) to iii) are conducted in the same vessel.

3. The method of claim 1 or claim 2, wherein the vessel is a multiwell plate such as a 96-, 384- or 1536-well plate.

4. The method of any preceding claim, wherein
a) steps i), ii) and iii) are each independently conducted at a substantially constant temperature; and/or
b) steps ii) and iii) are conducted at the same substantially constant temperature; and/or
c) steps i), ii) and iii) are conducted at the same substantially constant temperature; and/or
d) steps ii) and/or iii) of the method are conducted at ambient temperature, such as at a temperature of between approximately 16°C and 25°C.

5. The method of any preceding claim, wherein the assaying of step iii) detects the protein target in its folded state and/or its denatured state, such as unfolded or precipitated states.

6. The method of any of the preceding claims, wherein the assaying referred to in step iii) is carried out directly upon the denaturing mixture produced in step ii).

7. The method of any preceding claim, wherein the assaying is performed using: Homogeneous proximity assays or enzyme fragment complementation assay .

8. The method of any preceding claim, wherein the chemical denaturing agent comprises an acid, an alcohol and a ketone, optionally wherein the ratio of the acid, alcohol and ketone is approximately 0.1:50:50.

9. The method of any preceding claim, wherein the chemical denaturing agent comprises
a) a C1-C5 acid, such as a carboxylic acid like acetic acid, optionally wherein the acid is present in a denaturing mixture at a concentration of between about 0.01% and 0.2%; and/or
b) a C2-C5 alcohol, such as ethanol or propanol, optionally wherein the alcohol is present in a denaturing mixture at a concentration of between about 30% and 100%; and/or
c) a C2-C5 ketone, such as acetone or butanone, optionally wherein the ketone is present in a denaturing mixture at a concentration of between about 0% and 70%.

10. The method of any preceding claim, wherein the chemical denaturing agent is selected from the group consisting of:
• a mixture comprising: acetic acid; and ethanol; and acetone;
• a mixture comprising: acetic acid; and ethanol; and butanone;
• a mixture comprising: acetic acid; and propanol; and butanone;
• a mixture comprising: acetic acid; and ethanol;
• a mixture comprising: acetic acid; and propanol;
• a mixture comprising: formic acid; and ethanol; and acetone;
• a mixture comprising: formic acid; and ethanol; and butanone;
• a mixture comprising: formic acid; and propanol; and butanone;
• a mixture comprising: formic acid; and ethanol;
• a mixture comprising: formic acid; and propanol;
• a mixture comprising: propanoic acid; and ethanol; and acetone;
• a mixture comprising: propanoic acid; and ethanol; and butanone;
• a mixture comprising: propanoic acid; and propanol; and butanone;
• a mixture comprising: propanoic acid; and ethanol;
• a mixture comprising: propanoic acid; and propanol;
• a mixture comprising: butanoic acid; and ethanol; and acetone;
• a mixture comprising: butanoic acid; and ethanol; and butanone;
• a mixture comprising: butanoic acid; and propanol; and butanone;
• a mixture comprising: butanoic acid; and ethanol;
• a mixture comprising: butanoic acid; and propanol;
• a mixture comprising: pentanoic acid; and ethanol; and acetone;
• a mixture comprising: pentanoic acid; and ethanol; and butanone;
• a mixture comprising: pentanoic acid; and propanol; and butanone;
• a mixture comprising: pentanoic acid; and ethanol;
• a mixture comprising: pentanoic acid; and propanol;
• a mixture comprising: ethanol; and acetone;
• a mixture comprising: ethanol; and butanone;
• a mixture comprising: propanol; and acetone;
• a mixture comprising: propanol; and butanone;
• a denaturant solution comprising; ethanol alone; and
• a denaturant solution comprising; propanol alone.

11. The method of any preceding claim, wherein in step (ii) the target protein is contacted with range of denaturant agent in parallel, optionally wherein each distinct denaturant agent is in a separate well of a multiwell plate.

12. The method of claim 11, wherein the range of denaturant agent comprises Acetone/Ethanol/Acetic Acid (AEA), ethanol/acetic acid (EA), ethanol (E), butanone/propanol/acetic acid (BPA), propanol/acetic acid (PA) or propanol (P).

13. The method of any preceding claim, wherein the protein target is selected from the group consisting of: an enzyme including kinase, phosphatase, protease, hydrolase, dehydrogenase, synthase, lipase, ligase; A growth factor receptor, including a receptor tyrosine kinase; an intracellular proteins, such as Bcl family, nuclear proteins and mitochondrial proteins; a membrane protein, including GPCR, Ion Channel, Transporter, Integrin; and a secreted protein such as a cytokine, chemokine or growth factor.

14. The method of any preceding claim, wherein
a) the protein target is provided in step i) at a concentration of between 0.1 ng/ul and 5 µg/µl; and/or
b) the compound of interest is provided in step i) at a concentration of up to 100 mM; and/or
c) the protein target is exposed to the compound of interest for a period of at least 5 minutes, such as for a period of between 30 minutes and 24 hours; and/or
d) the protein target is provided in the denaturing mixture at a concentration of between 0.1 ng/ul and 5 µg/µl; and/or
e) the protein target and chemical denaturing agent are contacted with one another for a period of at least 5 minutes, such as for a period of between 30 minutes and 24 hours.

## Patentansprüche

1. Verfahren zum Identifizieren, ob ein Protein-Target und eine Verbindung von Interesse aneinander binden, wobei das Verfahren der Reihe nach folgende Schritte umfasst:
i) Aussetzen des Protein-Targets der Verbindung von Interesse über einen Zeitraum, der ausreicht, um eine Bindung der Verbindung von Interesse an das Protein-Target zu ermöglichen;
ii) Inkontaktbringen des Protein-Targets mit einem chemischen Denaturierungsmittel, um ein Denaturierungsgemisch herzustellen;
iii) Analysieren der Produkte des Denaturierungsgemischs, um den Anteil des Protein-Targets in seinem gefalteten Zustand und/oder den Anteil des Proteins in seinem denaturierten Zustand zu bestimmen;
wobei eine Veränderung des Anteils an Protein in seinem gefalteten Zustand und/oder des Anteils an Protein in seinem denaturierten Zustand im Vergleich zu einer geeigneten Kontrolle anzeigt, dass das Protein-Target und die Verbindung von Interesse aneinander binden, und wobei Schritt ii) des Verfahrens bei einer im Wesentlichen konstanten Temperatur durchgeführt wird; wobei Schritte ii) und iii) in demselben Behälter durchgeführt werden, und wobei das chemische Denaturierungsmittel mindestens eines von einer Säure, einem Alkohol und einem Keton umfasst.

2. Verfahren nach Anspruch 1, wobei jeder der Schritte i) bis iii) in demselben Behälter durchgeführt wird.

3. Verfahren nach Anspruch 1 oder Anspruch 2, wobei der Behälter eine Multiwell-Platte, wie etwa eine 96-, 384- oder 1536-Well-Platte ist.

4. Verfahren nach einem vorhergehenden Anspruch, wobei
a) Schritte i), ii) und iii) jeweils unabhängig bei einer im Wesentlichen konstanten Temperatur durchgeführt werden, und/oder
b) Schritte ii) und iii) bei der gleichen im Wesentlichen konstanten Temperatur durchgeführt werden, und/oder
c) Schritte i), ii) und iii) bei der gleichen im Wesentlichen konstanten Temperatur durchgeführt werden, und/oder
d) Schritte ii) und/oder iii) des Verfahrens bei Umgebungstemperatur, wie etwa bei einer Temperatur zwischen etwa 16 °C und 25 °C, durchgeführt werden.

5. Verfahren nach einem der vorhergehenden Ansprüche, wobei das Analysieren von Schritt iii) das Protein-Target in seinem gefalteten Zustand und/oder seinem denaturierten Zustand, wie etwa ungefaltete oder ausgefällte Zustände, detektiert.

6. Verfahren nach einem der vorhergehenden Ansprüche, wobei das in Schritt iii) genannte Analysieren direkt an dem in Schritt ii) hergestellten Denaturierungsgemisch durchgeführt wird.

7. Verfahren nach einem der vorhergehenden Ansprüche, wobei das Analysieren unter Verwendung von Homogenous Proximity Assays oder Enzymfragment-Komplementationsassay durchgeführt wird.

8. Verfahren nach einem der vorhergehenden Ansprüche, wobei das chemische Denaturierungsmittel eine Säure, einen Alkohol und ein Keton umfasst, wobei optional das Verhältnis der Säure, des Alkohols und des Ketons etwa 0,1:50:50 beträgt.

9. Verfahren nach einem der vorhergehenden Ansprüche, wobei das chemische Denaturierungsmittel umfasst
a) eine C1-C5-Säure, beispielsweise eine Carbonsäure wie etwa Essigsäure, wobei die Säure optional in einem Denaturierungsgemisch in einer Konzentration zwischen etwa 0,01 % und 0,2 % vorliegt, und/oder
b) einen C2-C5-Alkohol, beispielsweise Ethanol oder Propanol, wobei optional der Alkohol in einem Denaturierungsgemisch in einer Konzentration zwischen etwa 30 % und 100 % vorliegt, und/oder
c) ein C2-C5-Keton, beispielsweise Aceton oder Butanon, wobei optional das Keton in einem Denaturierungsgemisch in einer Konzentration zwischen etwa 0 % und 70 % vorliegt.

10. Verfahren nach einem der vorhergehenden Ansprüche, wobei das chemische Denaturierungsmittel ausgewählt ist aus der Gruppe bestehend aus:
• einem Gemisch, umfassend: Essigsäure und Ethanol und Aceton;
• einem Gemisch, umfassend: Essigsäure und Ethanol und Butanon;
• einem Gemisch, umfassend: Essigsäure und Propanol und Butanon;
• einem Gemisch, umfassend: Essigsäure und Ethanol;
• einem Gemisch, umfassend: Essigsäure und Propanol;
• einem Gemisch, umfassend: Ameisensäure und Ethanol und Aceton;
• einem Gemisch, umfassend: Ameisensäure und Ethanol und Butanon;
• einem Gemisch, umfassend: Ameisensäure und Propanol und Butanon;
• einem Gemisch, umfassend: Ameisensäure und Ethanol;
• einem Gemisch, umfassend: Ameisensäure und Propanol;
• einem Gemisch, umfassend: Propansäure und Ethanol und Aceton;
• einem Gemisch, umfassend: Propansäure und Ethanol und Butanon;
• einem Gemisch, umfassend: Propansäure und Propanol und Butanon;
• einem Gemisch, umfassend: Propansäure und Ethanol;
• einem Gemisch, umfassend: Propansäure und Propanol;
• einem Gemisch, umfassend: Butansäure und Ethanol und Aceton;
• einem Gemisch, umfassend: Butansäure und Ethanol und Butanon;
• einem Gemisch, umfassend: Butansäure und Propanol, und Butanon;
• einem Gemisch, umfassend: Butansäure und Ethanol;
• einem Gemisch, umfassend: Butansäure und Propanol;
• einem Gemisch, umfassend: Pentansäure und Ethanol, und Aceton;
• einem Gemisch, umfassend: Pentansäure und Ethanol, und Butanon;
• einem Gemisch, umfassend: Pentansäure und Propanol, und Butanon;
• einem Gemisch, umfassend: Pentansäure und Ethanol;
• einem Gemisch, umfassend: Pentansäure und Propanol;
• einem Gemisch, umfassend: Ethanol und Aceton;
• einem Gemisch, umfassend: Ethanol und Butanon;
• einem Gemisch, umfassend: Propanol und Aceton;
• einem Gemisch, umfassend: Propanol und Butanon;
• einer Denaturierungslösung, umfassend: Ethanol allein, und
• einer Denaturierungslösung, umfassend: Propanol allein.

11. Verfahren nach einem vorhergehenden Anspruch, wobei in Schritt (ii) das Zielprotein mit einer Auswahl von Denaturierungsmitteln parallel in Kontakt gebracht wird, wobei sich optional jedes unterschiedliche Denaturierungsmittel in einer separaten Vertiefung einer Multiwell-Platte befindet.

12. Verfahren nach Anspruch 11, wobei die Auswahl des Denaturierungsmittels Aceton/Ethanol/Essigsäure (AEA), Ethanol/Essigsäure (EA), Ethanol (E), Butanon/Propanol/Essigsäure (BPA), Propanol/Essigsäure (PA) oder Propanol (P) umfasst.

13. Verfahren nach einem vorhergehenden Anspruch, wobei das Protein-Target ausgewählt ist aus der Gruppe bestehend aus: einem Enzym, das Kinase, Phosphatase, Protease, Hydrolase, Dehydrogenase, Synthase, Lipase, Ligase beinhaltet; einem Wachstumsfaktorrezeptor, der eine Rezeptortyrosinkinase beinhaltet; einem intrazellulären Protein, wie etwa Bel-Familie, Kernproteine und mitochondriale Proteine; einem Membranprotein, das GPCR, Ionenkanal, Transporter, Integrin beinhaltet, und einem sekretierten Protein, beispielsweise einem Zytokin, Chemokin oder Wachstumsfaktor.

14. Verfahren nach einem vorhergehenden Anspruch, wobei
a) das Protein-Target in Schritt i) in einer Konzentration zwischen 0,1 ng/µl und 5 µg/µl bereitgestellt wird, und/oder
b) die Verbindung von Interesse in Schritt i) in einer Konzentration von bis zu 100 mM bereitgestellt wird, und/oder
c) das Protein-Target der Verbindung von Interesse über einen Zeitraum von mindestens 5 Minuten ausgesetzt wird, beispielsweise über einen Zeitraum zwischen 30 Minuten und 24 Stunden, und/oder
d) das Protein-Target in dem Denaturierungsgemisch in einer Konzentration zwischen 0,1 ng/µl und 5 µg/µl bereitgestellt wird, und/oder
e) das Protein-Target und das chemische Denaturierungsmittel über einen Zeitraum von mindestens 5 Minuten miteinander in Kontakt gebracht werden, wie etwa über einen Zeitraum zwischen 30 Minuten und 24 Stunden.

## Revendications

1. Procédé servant à identifier si une cible protéique et un composé d'intérêt se lient l'un à l'autre, le procédé comprenant, dans l'ordre, les étapes de :
i) exposition de la cible protéique au composé d'intérêt pendant un temps suffisant pour permettre la liaison du composé d'intérêt à la cible protéique ;
ii) mise en contact de la cible protéique avec un agent de dénaturation chimique pour produire un mélange de dénaturation ;
iii) dosage des produits du mélange de dénaturation pour déterminer la proportion de la cible protéique dans son état replié et/ou la proportion de protéine dans son état dénaturé ;
dans lequel un changement de la proportion de protéine dans son état replié et/ou de la proportion de protéine dans son état dénaturé par rapport à un témoin approprié indique que la cible protéique et le composé d'intérêt se lient l'un à l'autre, et dans lequel l'étape ii) du procédé est réalisée à une température sensiblement constante ; dans lequel les étapes ii) et iii) sont réalisées dans le même récipient ; et dans lequel l'agent de dénaturation chimique comprend au moins l'un parmi : un acide ; un alcool ; et une cétone.

2. Procédé de la revendication 1, dans lequel chacune des étapes i) à iii) est réalisée dans le même récipient.

3. Procédé de la revendication 1 ou la revendication 2, dans lequel le récipient est une plaque multipuits telle qu'une plaque à 96, 384 ou 1536 puits.

4. Procédé d'une quelconque revendication précédente, dans lequel
a) les étapes i), ii) et iii) sont réalisées chacune indépendamment à une température sensiblement constante ; et/ou
b) les étapes ii) et iii) sont réalisées à la même température sensiblement constante ; et/ou
c) les étapes i), ii) et iii) sont réalisées à la même température sensiblement constante ; et/ou
d) les étapes ii) et/ou iii) du procédé sont réalisées à température ambiante, telle qu'une température allant d'environ 16 °C à 25 °C.

5. Procédé d'une quelconque revendication précédente, dans lequel le dosage de l'étape iii) détecte la cible protéique dans son état replié et/ou son état dénaturé, tel que des états dépliés ou précipités.

6. Procédé de l'une quelconque des revendications précédentes, dans lequel le dosage mentionné à l'étape iii) est effectué directement sur le mélange de dénaturation produit à l'étape ii).

7. Procédé d'une quelconque revendication précédente, dans lequel le dosage est réalisé en utilisant : des essais de proximité homogène ou un essai de complémentation de fragments enzymatiques.

8. Procédé d'une quelconque revendication précédente, dans lequel l'agent de dénaturation chimique comprend un acide, un alcool et une cétone, éventuellement dans lequel le rapport entre l'acide, l'alcool et la cétone est d'environ 0,1:50:50.

9. Procédé d'une quelconque revendication précédente, dans lequel l'agent de dénaturation chimique comprend
a) un acide en C1-C5, tel qu'un acide carboxylique comme l'acide acétique, éventuellement ledit acide étant présent dans un mélange de dénaturation à une concentration allant d'environ 0,01 % à 0,2 % ; et/ou
b) un alcool en C2-C5, tel que l'éthanol ou le propanol, éventuellement ledit alcool étant présent dans un mélange de dénaturation à une concentration allant d'environ 30 % à 100 % ; et/ou
c) une cétone en C2-C5, telle que l'acétone ou la butanone, éventuellement ladite cétone étant présente dans un mélange de dénaturation à une concentration allant d'environ 0 % à 70 %.

10. Procédé d'une quelconque revendication précédente, dans lequel l'agent de dénaturation chimique est choisi dans le groupe constitué par :
• un mélange comprenant : de l'acide acétique ; et de l'éthanol ; et de l'acétone ;
• un mélange comprenant : de l'acide acétique ; et de l'éthanol ; et de la butanone ;
• un mélange comprenant : de l'acide acétique ; et du propanol ; et de la butanone ;
• un mélange comprenant : de l'acide acétique ; et de l'éthanol ;
• un mélange comprenant : de l'acide acétique ; et du propanol ;
• un mélange comprenant : de l'acide formique ; et de l'éthanol ; et de l'acétone ;
• un mélange comprenant : de l'acide formique ; et de l'éthanol ; et de la butanone ;
• un mélange comprenant : de l'acide formique ; et du propanol ; et de la butanone ;
• un mélange comprenant : de l'acide formique ; et de l'éthanol ;
• un mélange comprenant : de l'acide formique ; et du propanol ;
• un mélange comprenant : de l'acide propanoïque ; et de l'éthanol ; et de l'acétone ;
• un mélange comprenant : de l'acide propanoïque ; et de l'éthanol ; et de la butanone ;
• un mélange comprenant : de l'acide propanoïque ; et du propanol ; et de la butanone ;
• un mélange comprenant : de l'acide propanoïque ; et de l'éthanol ;
• un mélange comprenant : de l'acide propanoïque ; et du propanol ;
• un mélange comprenant : de l'acide butanoïque ; et de l'éthanol ; et de l'acétone ;
• un mélange comprenant : de l'acide butanoïque ; et de l'éthanol ; et de la butanone ;
• un mélange comprenant : de l'acide butanoïque ; et du propanol ; et de la butanone ;
• un mélange comprenant : de l'acide butanoïque ; et de l'éthanol ;
• un mélange comprenant : de l'acide butanoïque ; et du propanol ;
• un mélange comprenant : de l'acide pentanoïque ; et de l'éthanol ; et de l'acétone ;
• un mélange comprenant : de l'acide pentanoïque ; et de l'éthanol ; et de la butanone ;
• un mélange comprenant : de l'acide pentanoïque ; et du propanol ; et de la butanone ;
• un mélange comprenant : de l'acide pentanoïque ; et de l'éthanol ;
• un mélange comprenant : de l'acide pentanoïque ; et du propanol ;
• un mélange comprenant : de l'éthanol ; et de l'acétone ;
• un mélange comprenant : de l'éthanol ; et de la butanone ;
• un mélange comprenant : du propanol ; et de l'acétone ;
• un mélange comprenant : du propanol ; et de la butanone ;
• une solution de dénaturation comprenant ; de l'éthanol seul ; et
• une solution de dénaturation comprenant ; du propanol seul.

11. Procédé d'une quelconque revendication précédente, dans lequel à l'étape (ii), la protéine cible est mise en contact avec une gamme d'agents de dénaturation en parallèle, éventuellement dans lequel chaque agent de dénaturation distinct se trouve dans un puits séparé d'une plaque multipuits.

12. Procédé de la revendication 11, dans lequel la gamme d'agents de dénaturation comprend l'acétone/éthanol/acide acétique (AEA), l'éthanol/acide acétique (EA), l'éthanol (E), la butanone/propanol/acide acétique (BPA), le propanol/acide acétique (PA) ou le propanol (P).

13. Procédé d'une quelconque revendication précédente, dans lequel la cible protéique est choisie dans le groupe constitué par : une enzyme comprenant une kinase, une phosphatase, une protéase, une hydrolase, une déshydrogénase, une synthase, une lipase, une ligase ; un récepteur de facteur de croissance, comprenant une tyrosine kinase réceptrice ; des protéines intracellulaires, telle que la famille Bcl, des protéines nucléaires et des protéines mitochondriales ; une protéine membranaire, comprenant un GPCR, un canal ionique, un transporteur, une intégrine ; et une protéine sécrétée telle qu'une cytokine, une chimiokine ou un facteur de croissance.

14. Procédé d'une quelconque revendication précédente, dans lequel
a) la cible protéique est fournie à l'étape i) à une concentration allant de 0,1 ng/µl à 5 µg/µl ; et/ou
b) le composé d'intérêt est fourni à l'étape i) à une concentration allant jusqu'à 100 mM ; et/ou
c) la cible protéique est exposée au composé d'intérêt pendant une période d'au moins 5 minutes, par exemple pendant une période allant de 30 minutes à 24 heures ; et/ou
d) la cible protéique est fournie dans le mélange de dénaturation à une concentration allant de 0,1 ng/µl à 5 µg/µl ; et/ou
e) la cible protéique et l'agent de dénaturation chimique sont mis en contact l'un avec l'autre pendant une période d'au moins 5 minutes, par exemple pendant une période allant de 30 minutes à 24 heures.
